# EUROPEAN PATENT SPECIFICATION

(11) **EP 2 114 993 B1**
(45) Date of publication and mention of the grant of the patent: **29.08.2012**
(21) Application number: 08701434.6
(22) Date of filing: 11.01.2008
(51) Int. Cl.: C07K 14/285, A61K 39/00, C07K 14/47

(54) **VACCINE**
IMPSTOFF
VACCIN

(30) Priority: 15.01.2007 GB 0700760; 23.01.2007 GB 0701262
(43) Date of publication of application: 11.11.2009
(73) Proprietor: GlaxoSmithKline Biologicals SA, 1330 Rixensart (BE)
(72) Inventor: BLAIS, Normand, Laval, H7V 3S8 (CA); MARTIN, Denis, Laval, H7V 3S8 (CA); PALMANTIER, Remi, M., Laval, H7V 3S8 (CA)
(74) Representative: Lovatt, Victoria Jayne
(86) International application number: PCT/EP2008/050290
(87) International publication number: WO 2008/087102

(56) References cited:
- EP-A- 1 584 685
- WO-A-00/50077
- WO-A-01/00232
- WO-A-99/10375
- WO-A-99/40188
- US-A1- 2002 182 221
- HALLEZ SOPHIE ET AL: "Phase I/II trial of immunogenicity of a human papillomavirus (HPV) type 16 E7 protein-based vaccine in women with oncogenic HPV-positive cervical intraepithelial neoplasia." CANCER IMMUNOLOGY, IMMUNOTHERAPY : CII JUL 2004, vol. 53, no. 7, July 2004 (2004-07), pages 642-650, XP002477749 ISSN: 0340-7004

### SEQUENCE LISTING

<110> GlaxoSmithKline Biologicals SA
<120> Vaccine
<130> VB62293
<150> 0700760.2
   <151> 2007-01-15
<150> 0701262.8
   <151> 2007-01-23
<160> 45
<170> FastSEQ for Windows Version 4.0
<210> 1
   <211> 1887
   <212> DNA
   <213> Artificial Sequence
<220>
   <223> MDP- 20-127 - Protein D - PRAME - TSGHHHHHH (plasmid TCMP14)
<400> 1
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accatggaac gaaggcgttt gtggggttcc 360
   attcagagcc gatacatcag catgagtgtg tggacaagcc cacggagact tgtggagctg 420
   gcagggcaga gcctgctgaa ggatgaggcc ctggccattg ccgccctgga gttgctgccc 480
   agggagctct tcccgccact cttcatggca gcctttgacg ggagacacag ccagaccctg 540
   aaggcaatgg tgcaggcctg gcccttcacc tgcctccctc tgggagtgct gatgaaggga 600
   caacatcttc acctggagac cttcaaagct gtgcttgatg gacttgatgt gctccttgcc 660
   caggaggttc gccccaggag gtggaaactt caagtgctgg atttacggaa gaactctcat 720
   caggacttct ggactgtatg gtctggaaac agggccagtc tgtactcatt tccagagcca 780
   gaagcagctc agcccatgac aaagaagcga aaagtagatg gtttgagcac agaggcagag 840
   cagcccttca ttccagtaga ggtgctcgta gacctgttcc tcaaggaagg tgcctgtgat 900
   gaattgttct cctacctcat tgagaaagtg aagcgaaaga aaaatgtact acgcctgtgc 960
   tgtaagaagc tgaagatttt tgcaatgccc atgcaggata tcaagatgat cctgaaaatg 1020
   gtgcagctgg actctattga agatttggaa gtgacttgta cctggaagct acccaccttg 1080
   gcgaaatttt ctccttacct gggccagatg attaatctgc gtagactcct cctctcccac 1140
   atccatgcat cttcctacat ttccccggag aaggaagagc agtatatcgc ccagttcacc 1200
   tctcagttcc tcagtctgca gtgcctgcag gctctctatg tggactcttt atttttcctt 1260
   agaggccgcc tggatcagtt gctcaggcac gtgatgaacc ccttggaaac cctctcaata 1320
   actaactgcc ggctttcgga aggggatgtg atgcatctgt cccagagtcc cagcgtcagt 1380
   cagctaagtg tcctgagtct aagtggggtc atgctgaccg atgtaagtcc cgagcccctc 1440
   caagctctgc tggagagagc ctctgccacc ctccaggacc tggtctttga tgagtgtggg 1500
   atcacggatg atcagctcct tgccctcctg ccttccctga gccactgctc ccagcttaca 1560
   accttaagct tctacgggaa ttccatctcc atatctgcct tgcagagtct cctgcagcac 1620
   ctcatcgggc tgagcaatct gacccacgtg ctgtatcctg tccccctgga gagttatgag 1680
   gacatccatg gtaccctcca cctggagagg cttgcctatc tgcatgccag gctcagggag 1740
   ttgctgtgtg agttggggcg gcccagcatg gtctggctta gtgccaaccc ctgtcctcac 1800
   tgtggggaca gaaccttcta tgacccggag cccatcctgt gcccctgttt catgcctaac 1860
   actagtggcc accatcacca tcaccat                                    1887
<210> 2
   <211> 629
   <212> PRT
   <213> Artificial Sequence
<220>
   <223> MDP- 20-127 - Protein D - PRAME - TSGHHHHHH (plasmid TCMP14)
<400> 2
<210> 3
   <211> 1860
   <212> DNA
   <213> Artificial Sequence
<220>
   <223> MDP- 20-127 - Protein D - PRAME - no His tail (plasmid TCMP14)
<400> 3
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accatggaac gaaggcgttt gtggggttcc 360
   attcagagcc gatacatcag catgagtgtg tggacaagcc cacggagact tgtggagctg 420
   gcagggcaga gcctgctgaa ggatgaggcc ctggccattg ccgccctgga gttgctgccc 480
   agggagctct tcccgccact cttcatggca gcctttgacg ggagacacag ccagaccctg 540
   aaggcaatgg tgcaggcctg gcccttcacc tgcctccctc tgggagtgct gatgaaggga 600
   caacatcttc acctggagac cttcaaagct gtgcttgatg gacttgatgt gctccttgcc 660
   caggaggttc gccccaggag gtggaaactt caagtgctgg atttacggaa gaactctcat 720
   caggacttct ggactgtatg gtctggaaac agggccagtc tgtactcatt tccagagcca 780
   gaagcagctc agcccatgac aaagaagcga aaagtagatg gtttgagcac agaggcagag 840
   cagcccttca ttccagtaga ggtgctcgta gacctgttcc tcaaggaagg tgcctgtgat 900
   gaattgttct cctacctcat tgagaaagtg aagcgaaaga aaaatgtact acgcctgtgc 960
   tgtaagaagc tgaagatttt tgcaatgccc atgcaggata tcaagatgat cctgaaaatg 1020
   gtgcagctgg actctattga agatttggaa gtgacttgta cctggaagct acccaccttg 1080
   gcgaaatttt ctccttacct gggccagatg attaatctgc gtagactcct cctctcccac 1140
   atccatgcat cttcctacat ttccccggag aaggaagagc agtatatcgc ccagttcacc 1200
   tctcagttcc tcagtctgca gtgcctgcag gctctctatg tggactcttt atttttcctt 1260
   agaggccgcc tggatcagtt gctcaggcac gtgatgaacc ccttggaaac cctctcaata 1320
   actaactgcc ggctttcgga aggggatgtg atgcatctgt cccagagtcc cagcgtcagt 1380
   cagctaagtg tcctgagtct aagtggggtc atgctgaccg atgtaagtcc cgagcccctc 1440
   caagctctgc tggagagagc ctctgccacc ctccaggacc tggtctttga tgagtgtggg 1500
   atcacggatg atcagctcct tgccctcctg ccttccctga gccactgctc ccagcttaca 1560
   accttaagct tctacgggaa ttccatctcc atatctgcct tgcagagtct cctgcagcac 1620
   ctcatcgggc tgagcaatct gacccacgtg ctgtatcctg tccccctgga gagttatgag 1680
   gacatccatg gtaccctcca cctggagagg cttgcctatc tgcatgccag gctcagggag 1740
   ttgctgtgtg agttggggcg gcccagcatg gtctggctta gtgccaaccc ctgtcctcac 1800
   tgtggggaca gaaccttcta tgacccggag cccatcctgt gcccctgttt catgcctaac 1860
<210> 4
   <211> 620
   <212> PRT
   <213> Artificial Sequence
<220>
   <223> MDP- 20-127 - Protein D - PRAME - no His tail (plasmid TCMP14)
<400> 4
   Met Asp Pro Ser Ser His Ser Ser Asn Met Ala Asn Thr Gln Met Lys
   1               5                   10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Met
   100 105 110
   Glu Arg Arg Arg Leu Trp Gly Ser Ile Gln Ser Arg Tyr Ile Ser Met
   115 120 125
   Ser Val Trp Thr Ser Pro Arg Arg Leu Val Glu Leu Ala Gly Gln Ser
   130 135 140
   Leu Leu Lys Asp Glu Ala Leu Ala Ile Ala Ala Leu Glu Leu Leu Pro
   145 150 155 160
   Arg Glu Leu Phe Pro Pro Leu Phe Met Ala Ala Phe Asp Gly Arg His
   165 170 175
   Ser Gln Thr Leu Lys Ala Met Val Gln Ala Trp Pro Phe Thr Cys Leu
   180 185 190
   Pro Leu Gly Val Leu Met Lys Gly Gln His Leu His Leu Glu Thr Phe
   195 200 205
   Lys Ala Val Leu Asp Gly Leu Asp Val Leu Leu Ala Gln Glu Val Arg
   210 215 220
   Pro Arg Arg Trp Lys Leu Gln Val Leu Asp Leu Arg Lys Asn Ser His
   225 230 235 240
   Gln Asp Phe Trp Thr Val Trp Ser Gly Asn Arg Ala Ser Leu Tyr Ser
   245 250 255
   Phe Pro Glu Pro Glu Ala Ala Gln Pro Met Thr Lys Lys Arg Lys Val
   260 265 270
   Asp Gly Leu Ser Thr Glu Ala Glu Gln Pro Phe Ile Pro Val Glu Val
   275 280 285
   Leu Val Asp Leu Phe Leu Lys Glu Gly Ala Cys Asp Glu Leu Phe Ser
   290 295 300
   Tyr Leu Ile Glu Lys Val Lys Arg Lys Lys Asn Val Leu Arg Leu Cys
   305 310 315 320
   Cys Lys Lys Leu Lys Ile Phe Ala Met Pro Met Gln Asp Ile Lys Met
   325 330 335
   Ile Leu Lys Met Val Gln Leu Asp Ser Ile Glu Asp Leu Glu Val Thr
   340 345 350
   Cys Thr Trp Lys Leu Pro Thr Leu Ala Lys Phe Ser Pro Tyr Leu Gly
   355 360 365
   Gln Met Ile Asn Leu Arg Arg Leu Leu Leu Ser His Ile His Ala Ser
   370 375 380
   Ser Tyr Ile Ser Pro Glu Lys Glu Glu Gln Tyr Ile Ala Gln Phe Thr
   385 390 395 400
   Ser Gln Phe Leu Ser Leu Gln Cys Leu Gln Ala Leu Tyr Val Asp Ser
   405 410 415
   Leu Phe Phe Leu Arg Gly Arg Leu Asp Gln Leu Leu Arg His Val Met
   420 425 430
   Asn Pro Leu Glu Thr Leu Ser Ile Thr Asn Cys Arg Leu Ser Glu Gly
   435 440 445
   Asp Val Met His Leu Ser Gln Ser Pro Ser Val Ser Gln Leu Ser Val
       450                 455                 460
   Leu Ser Leu Ser Gly Val Met Leu Thr Asp Val Ser Pro Glu Pro Leu
   465                 470                 475                 480
   Gln Ala Leu Leu Glu Arg Ala Ser Ala Thr Leu Gln Asp Leu Val Phe
                   485                 490                 495
   Asp Glu Cys Gly Ile Thr Asp Asp Gln Leu Leu Ala Leu Leu Pro Ser
               500                 505                 510
   Leu Ser His Cys Ser Gln Leu Thr Thr Leu Ser Phe Tyr Gly Asn Ser
           515                 520                 525
   Ile Ser Ile Ser Ala Leu Gln Ser Leu Leu Gln His Leu Ile Gly Leu
       530                 535                 540
   Ser Asn Leu Thr His Val Leu Tyr Pro Val Pro Leu Glu Ser Tyr Glu
   545                 550                 555                 560
   Asp Ile His Gly Thr Leu His Leu Glu Arg Leu Ala Tyr Leu His Ala
                   565                 570                 575
   Arg Leu Arg Glu Leu Leu Cys Glu Leu Gly Arg Pro Ser Met Val Trp
               580                 585                 590
   Leu Ser Ala Asn Pro Cys Pro His Cys Gly Asp Arg Thr Phe Tyr Asp
           595                 600                 605
   Pro Glu Pro Ile Leu Cys Pro Cys Phe Met Pro Asn
       610                 615                 620
<210> 5
   <211> 1884
   <212> DNA
   <213> Artificial Sequence
<220>
   <223> MDP- 20-127 - Protein D - PRAME - LEHHHHHH (plasmid pET21)
<400> 5
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accatggaac gaaggcgttt gtggggttcc 360
   attcagagcc gatacatcag catgagtgtg tggacaagcc cacggagact tgtggagctg 420
   gcagggcaga gcctgctgaa ggatgaggcc ctggccattg ccgccctgga gttgctgccc 480
   agggagctct tcccgccact cttcatggca gcctttgacg ggagacacag ccagaccctg 540
   aaggcaatgg tgcaggcctg gcccttcacc tgcctccctc tgggagtgct gatgaaggga 600
   caacatcttc acctggagac cttcaaagct gtgcttgatg gacttgatgt gctccttgcc 660
   caggaggttc gccccaggag gtggaaactt caagtgctgg atttacggaa gaactctcat 720
   caggacttct ggactgtatg gtctggaaac agggccagtc tgtactcatt tccagagcca 780
   gaagcagctc agcccatgac aaagaagcga aaagtagatg gtttgagcac agaggcagag 840
   cagcccttca ttccagtaga ggtgctcgta gacctgttcc tcaaggaagg tgcctgtgat 900
   gaattgttct cctacctcat tgagaaagtg aagcgaaaga aaaatgtact acgcctgtgc 960
   tgtaagaagc tgaagatttt tgcaatgccc atgcaggata tcaagatgat cctgaaaatg 1020
   gtgcagctgg actctattga agatttggaa gtgacttgta cctggaagct acccaccttg 1080
   gcgaaatttt ctccttacct gggccagatg attaatctgc gtagactcct cctctcccac 1140
   atccatgcat cttcctacat ttccccggag aaggaagagc agtatatcgc ccagttcacc 1200
   tctcagttcc tcagtctgca gtgcctgcag gctctctatg tggactcttt atttttcctt 1260
   agaggccgcc tggatcagtt gctcaggcac gtgatgaacc ccttggaaac cctctcaata 1320
   actaactgcc ggctttcgga aggggatgtg atgcatctgt cccagagtcc cagcgtcagt 1380
   cagctaagtg tcctgagtct aagtggggtc atgctgaccg atgtaagtcc cgagcccctc 1440
   caagctctgc tggagagagc ctctgccacc ctccaggacc tggtctttga tgagtgtggg 1500
   atcacggatg atcagctcct tgccctcctg ccttccctga gccactgctc ccagcttaca 1560
   accttaagct tctacgggaa ttccatctcc atatctgcct tgcagagtct cctgcagcac 1620
   ctcatcgggc tgagcaatct gacccacgtg ctgtatcctg tccccctgga gagttatgag 1680
   gacatccatg gtaccctcca cctggagagg cttgcctatc tgcatgccag gctcagggag 1740
   ttgctgtgtg agttggggcg gcccagcatg gtctggctta gtgccaaccc ctgtcctcac 1800
   tgtggggaca gaaccttcta tgacccggag cccatcctgt gcccctgttt catgcctaac 1860
   ctcgagcacc accaccacca ccac                                        1884
<210> 6
   <211> 628
   <212> PRT
   <213> Artificial Sequence
<220>
   <223> MDP- 20-127 - Protein D - PRAME - LEHHHHHH (plasmid pET21)
<400> 6
   Met Asp Pro Ser Ser His Ser Ser Asn Met Ala Asn Thr Gln Met Lys
   1               5                   10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
   85 90 95
   Leu Lys Glu Ile Gln Ser Leu Glu Met Thr Glu Asn Phe Glu Thr Met
   100 105 110
   Glu Arg Arg Arg Leu Trp Gly Ser Ile Gln Ser Arg Tyr Ile Ser Met
   115 120 125
   Ser Val Trp Thr Ser Pro Arg Arg Leu Val Glu Leu Ala Gly Gln Ser
   130 135 140
   Leu Leu Lys Asp Glu Ala Leu Ala Ile Ala Ala Leu Glu Leu Leu Pro
   145 150 155 160
   Arg Glu Leu Phe Pro Pro Leu Phe Met Ala Ala Phe Asp Gly Arg His
   165 170 175
   Ser Gln Thr Leu Lys Ala Met Val Gln Ala Trp Pro Phe Thr Cys Leu
   180 185 190
   Pro Leu Gly Val Leu Met Lys Gly Gln His Leu His Leu Glu Thr Phe
   195 200 205
   Lys Ala Val Leu Asp Gly Leu Asp Val Leu Leu Ala Gln Glu Val Arg
   210 215 220
   Pro Arg Arg Trp Lys Leu Gln Val Leu Asp Leu Arg Lys Asn Ser His
   225 230 235 240
   Gln Asp Phe Trp Thr Val Trp Ser Gly Asn Arg Ala Ser Leu Tyr Ser
   245 250 255
   Phe Pro Glu Pro Glu Ala Ala Gln Pro Met Thr Lys Lys Arg Lys Val
   260 265 270
   Asp Gly Leu Ser Thr Glu Ala Glu Gln Pro Phe Ile Pro Val Glu Val
   275 280 285
   Leu Val Asp Leu Phe Leu Lys Glu Gly Ala Cys Asp Glu Leu Phe Ser
   290 295 300
   Tyr Leu Ile Glu Lys Val Lys Arg Lys Lys Asn Val Leu Arg Leu Cys
   305 310 315 320
   Cys Lys Lys Leu Lys Ile Phe Ala Met Pro Met Gln Asp Ile Lys Met
   325 330 335
   Ile Leu Lys Met Val Gln Leu Asp Ser Ile Glu Asp Leu Glu Val Thr
   340 345 350
   Cys Thr Trp Lys Leu Pro Thr Leu Ala Lys Phe Ser Pro Tyr Leu Gly
   355 360 365
   Gln Met Ile Asn Leu Arg Arg Leu Leu Leu Ser His Ile His Ala Ser
   370 375 380
   Ser Tyr Ile Ser Pro Glu Lys Glu Glu Gln Tyr Ile Ala Gln Phe Thr
   385 390 395 400
   Ser Gln Phe Leu Ser Leu Gln Cys Leu Gln Ala Leu Tyr Val Asp Ser
   405 410 415
   Leu Phe Phe Leu Arg Gly Arg Leu Asp Gln Leu Leu Arg His Val Met
   420 425 430
   Asn Pro Leu Glu Thr Leu Ser Ile Thr Asn Cys Arg Leu Ser Glu Gly
           435                 440                 445
   Asp Val Met His Leu Ser Gln Ser Pro Ser Val Ser Gln Leu Ser Val
       450                 455                 460
   Leu Ser Leu Ser Gly Val Met Leu Thr Asp Val Ser Pro Glu Pro Leu
   465                 470                 475                 480
   Gln Ala Leu Leu Glu Arg Ala Ser Ala Thr Leu Gln Asp Leu Val Phe
                   485                 490                 495
   Asp Glu Cys Gly Ile Thr Asp Asp Gln Leu Leu Ala Leu Leu Pro Ser
               500                 505                 510
   Leu Ser His Cys Ser Gln Leu Thr Thr Leu Ser Phe Tyr Gly Asn Ser
           515                 520                 525
   Ile Ser Ile Ser Ala Leu Gln Ser Leu Leu Gln His Leu Ile Gly Leu
       530                 535                 540
   Ser Asn Leu Thr His Val Leu Tyr Pro Val Pro Leu Glu Ser Tyr Glu
   545                 550                 555                 560
   Asp Ile His Gly Thr Leu His Leu Glu Arg Leu Ala Tyr Leu His Ala
                   565                 570                 575
   Arg Leu Arg Glu Leu Leu Cys Glu Leu Gly Arg Pro Ser Met Val Trp
               580                 585                 590
   Leu Ser Ala Asn Pro Cys Pro His Cys Gly Asp Arg Thr Phe Tyr Asp
           595                 600                 605
   Pro Glu Pro Ile Leu Cys Pro Cys Phe Met Pro Asn Leu Glu His His
       610                 615                 620
   His His His His
   625
<210> 7
   <211> 1860
   <212> DNA
   <213> Artificial Sequence
<220>
   <223> MDP- 20-127 - Protein D - PRAME - no His tail (plasmid pET21)
<400> 7
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accatggaac gaaggcgttt gtggggttcc 360
   attcagagcc gatacatcag catgagtgtg tggacaagcc cacggagact tgtggagctg 420
   gcagggcaga gcctgctgaa ggatgaggcc ctggccattg ccgccctgga gttgctgccc 480
   agggagctct tcccgccact cttcatggca gcctttgacg ggagacacag ccagaccctg 540
   aaggcaatgg tgcaggcctg gcccttcacc tgcctccctc tgggagtgct gatgaaggga 600
   caacatcttc acctggagac cttcaaagct gtgcttgatg gacttgatgt gctccttgcc 660
   caggaggttc gccccaggag gtggaaactt caagtgctgg atttacggaa gaactctcat 720
   caggacttct ggactgtatg gtctggaaac agggccagtc tgtactcatt tccagagcca 780
   gaagcagctc agcccatgac aaagaagcga aaagtagatg gtttgagcac agaggcagag 840
   cagcccttca ttccagtaga ggtgctcgta gacctgttcc tcaaggaagg tgcctgtgat 900
   gaattgttct cctacctcat tgagaaagtg aagcgaaaga aaaatgtact acgcctgtgc 960
   tgtaagaagc tgaagatttt tgcaatgccc atgcaggata tcaagatgat cctgaaaatg 1020
   gtgcagctgg actctattga agatttggaa gtgacttgta cctggaagct acccaccttg 1080
   gcgaaatttt ctccttacct gggccagatg attaatctgc gtagactcct cctctcccac 1140
   atccatgcat cttcctacat ttccccggag aaggaagagc agtatatcgc ccagttcacc 1200
   tctcagttcc tcagtctgca gtgcctgcag gctctctatg tggactcttt atttttcctt 1260
   agaggccgcc tggatcagtt gctcaggcac gtgatgaacc ccttggaaac cctctcaata 1320
   actaactgcc ggctttcgga aggggatgtg atgcatctgt cccagagtcc cagcgtcagt 1380
   cagctaagtg tcctgagtct aagtggggtc atgctgaccg atgtaagtcc cgagcccctc 1440
   caagctctgc tggagagagc ctctgccacc ctccaggacc tggtctttga tgagtgtggg 1500
   atcacggatg atcagctcct tgccctcctg ccttccctga gccactgctc ccagcttaca 1560
   accttaagct tctacgggaa ttccatctcc atatctgcct tgcagagtct cctgcagcac 1620
   ctcatcgggc tgagcaatct gacccacgtg ctgtatcctg tccccctgga gagttatgag 1680
   gacatccatg gtaccctcca cctggagagg cttgcctatc tgcatgccag gctcagggag 1740
   ttgctgtgtg agttggggcg gcccagcatg gtctggctta gtgccaaccc ctgtcctcac 1800
   tgtggggaca gaaccttcta tgacccggag cccatcctgt gcccctgttt catgcctaac 1860
<210> 8
   <211> 620
   <212> PRT
   <213> Artificial Sequence
<220>
   <223> MDP- 20-127 - Protein D - PRAME - no His tail (plasmid pET21)
<400> 8
   Met Asp Pro Ser Ser His Ser Ser Asn Met Ala Asn Thr Gln Met Lys
    1               5                  10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
       50                  55                  60
   Ile His Asp His Phe Leu Asp Gly Leu Thr Asp Val Ala Lys Lys Phe
   65                  70                  75                  80
   Pro His Arg His Arg Lys Asp Gly Arg Tyr Tyr Val Ile Asp Phe Thr
                   85                  90                  95
   Leu Lys Glu Ile Gln Ser Leu Glu Met Thr Glu Asn Phe Glu Thr Met
               100                 105                 110
   Glu Arg Arg Arg Leu Trp Gly Ser Ile Gln Ser Arg Tyr Ile Ser Met
           115                 120                 125
   Ser Val Trp Thr Ser Pro Arg Arg Leu Val Glu Leu Ala Gly Gln Ser
       130                 135                 140
   Leu Leu Lys Asp Glu Ala Leu Ala Ile Ala Ala Leu Glu Leu Leu Pro
   145                 150                 155                 160
   Arg Glu Leu Phe Pro Pro Leu Phe Met Ala Ala Phe Asp Gly Arg His
                   165                 170                 175
   Ser Gln Thr Leu Lys Ala Met Val Gln Ala Trp Pro Phe Thr Cys Leu
               180                 185                 190
   Pro Leu Gly Val Leu Met Lys Gly Gln His Leu His Leu Glu Thr Phe
           195                 200                 205
   Lys Ala Val Leu Asp Gly Leu Asp Val Leu Leu Ala Gln Glu Val Arg
       210                 215                 220
   Pro Arg Arg Trp Lys Leu Gln Val Leu Asp Leu Arg Lys Asn Ser His
   225                 230                 235                 240
   Gln Asp Phe Trp Thr Val Trp Ser Gly Asn Arg Ala Ser Leu Tyr Ser
                   245                 250                 255
   Phe Pro Glu Pro Glu Ala Ala Gln Pro Met Thr Lys Lys Arg Lys Val
               260                 265                 270
   Asp Gly Leu Ser Thr Glu Ala Glu Gln Pro Phe Ile Pro Val Glu Val
           275                 280                 285
   Leu Val Asp Leu Phe Leu Lys Glu Gly Ala Cys Asp Glu Leu Phe Ser
       290                 295                 300
   Tyr Leu Ile Glu Lys Val Lys Arg Lys Lys Asn Val Leu Arg Leu Cys
   305                 310                 315                 320
   Cys Lys Lys Leu Lys Ile Phe Ala Met Pro Met Gln Asp Ile Lys Met
                   325                 330                 335
   Ile Leu Lys Met Val Gln Leu Asp Ser Ile Glu Asp Leu Glu Val Thr
               340                 345                 350
   Cys Thr Trp Lys Leu Pro Thr Leu Ala Lys Phe Ser Pro Tyr Leu Gly
           355                 360                 365
   Gln Met Ile Asn Leu Arg Arg Leu Leu Leu Ser His Ile His Ala Ser
       370                 375                 380
   Ser Tyr Ile Ser Pro Glu Lys Glu Glu Gln Tyr Ile Ala Gln Phe Thr
   385                 390                 395                 400
   Ser Gln Phe Leu Ser Leu Gln Cys Leu Gln Ala Leu Tyr Val Asp Ser
                   405                 410                 415
   Leu Phe Phe Leu Arg Gly Arg Leu Asp Gln Leu Leu Arg His Val Met
               420                 425                 430
   Asn Pro Leu Glu Thr Leu Ser Ile Thr Asn Cys Arg Leu Ser Glu Gly
           435                 440                 445
   Asp Val Met His Leu Ser Gln Ser Pro Ser Val Ser Gln Leu Ser Val
       450                 455                 460
   Leu Ser Leu Ser Gly Val Met Leu Thr Asp Val Ser Pro Glu Pro Leu
   465                 470                 475                 480
   Gln Ala Leu Leu Glu Arg Ala Ser Ala Thr Leu Gln Asp Leu Val Phe
                   485                 490                 495
   Asp Glu Cys Gly Ile Thr Asp Asp Gln Leu Leu Ala Leu Leu Pro Ser
               500                 505                 510
   Leu Ser His Cys Ser Gln Leu Thr Thr Leu Ser Phe Tyr Gly Asn Ser
           515                 520                 525
   Ile Ser Ile Ser Ala Leu Gln Ser Leu Leu Gln His Leu Ile Gly Leu
       530                 535                 540
   Ser Asn Leu Thr His Val Leu Tyr Pro Val Pro Leu Glu Ser Tyr Glu
   545                 550                 555                 560
   Asp Ile His Gly Thr Leu His Leu Glu Arg Leu Ala Tyr Leu His Ala
                   565                 570                 575
   Arg Leu Arg Glu Leu Leu Cys Glu Leu Gly Arg Pro Ser Met Val Trp
               580                 585                 590
   Leu Ser Ala Asn Pro Cys Pro His Cys Gly Asp Arg Thr Phe Tyr Asp
           595                 600                 605
   Pro Glu Pro Ile Leu Cys Pro Cys Phe Met Pro Asn
       610                 615                 620
<210> 9
   <211> 1878
   <212> DNA
   <213> Artificial Sequence
<220>
   <223> MDP- 20-127 - Protein D - PRAME - HHHHHH (plasmid pET26)
<400> 9
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accatggaac gtcgtcgtct gtggggcagc 360
   attcagagcc gttatattag catgagcgtg tggaccagcc cgcgtcgtct ggttgagctg 420
   gccggccaga gcctgctgaa agatgaagcg ctggccattg cggcgctgga gctgctgccg 480
   cgtgagctgt ttccgccgct gtttatggcg gcgtttgatg gccgtcatag ccagaccctg 540
   aaagcgatgg tgcaggcgtg gccgtttacc tgtctgccgc tgggcgtgct gatgaaaggc 600
   cagcatctgc atctggaaac ctttaaagcg gtgctggatg gcctggatgt gctgctggcc 660
   caggaagttc gtccgcgtcg ttggaaactg caagtgctgg atctgcgtaa aaacagccat 720
   caggattttt ggaccgtgtg gagcggcaat cgtgcgagcc tgtatagctt tccggaaccg 780
   gaagcggcgc agccgatgac caaaaaacgt aaagtggatg gcctgagcac cgaagcggaa 840
   cagccgttta ttccggtgga agtgctggtt gacctgtttc tgaaagaagg cgcctgcgac 900
   gagctgttta gctatctgat cgaaaaagtg aaacgcaaaa aaaacgtgct gcgtctgtgc 960
   tgcaaaaaac tgaaaatctt cgcgatgccg atgcaggata ttaaaatgat cctgaaaatg 1020
   gtgcagctgg atagcattga ggacctggaa gtgacctgca cctggaaact gccgaccctg 1080
   gccaaattta gcccgtatct gggccagatg attaacctgc gtcgtctgct gctgtctcat 1140
   attcatgcga gcagctatat tagcccggaa aaagaagaac agtatatcgc gcagtttacc 1200
   agccagtttc tgagcctgca atgcctgcaa gcgctgtatg tggatagcct gttttttctg 1260
   cgtggccgtc tggatcagct gctgcgtcat gtgatgaatc cgctggaaac cctgagcatt 1320
   accaactgcc gtctgagcga aggcgatgtg atgcatctga gccagagccc gagcgttagc 1380
   cagctgtctg ttctgagcct gagcggcgtg atgctgaccg atgtgagccc ggaaccgctg 1440
   caagccctgc tggaacgtgc gagcgcgacc ctgcaagacc tggtgtttga tgaatgcggc 1500
   attaccgatg atcagctgct ggccctgctg ccgagcctga gccattgcag ccagctgacc 1560
   accctgagct tttatggcaa cagcattagc attagcgcgc tgcaaagcct gctgcaacat 1620
   ctgattggcc tgagcaacct gacccatgtg ctgtatccgg tgccgctgga aagctatgaa 1680
   gatattcatg gcaccctgca tctggaacgt ctggcctatc tgcacgcgcg tctgcgtgag 1740
   ctgctgtgcg agctgggccg tccgagcatg gtttggctgt ctgcgaatcc gtgcccgcat 1800
   tgcggcgatc gtacctttta tgatccggaa ccgattctgt gcccgtgctt tatgccgaac 1860
   caccaccacc accaccac                                                  1878
<210> 10
   <211> 626
   <212> PRT
   <213> Artificial Sequence
<220>
   <223> MDP- 20-127 - Protein D - PRAME - HHHHHH (plasmid pET26)
<400> 10
   Met Asp Pro Ser Ser His Ser Ser Asn Met Ala Asn Thr Gln Met Lys
   1               5                   10                  15
   Ser Asp Lys Ile Ile Ile Ala His Arg Gly Ala Ser Gly Tyr Leu Pro
               20                  25                  30
   Glu His Thr Leu Glu Ser Lys Ala Leu Ala Phe Ala Gln Gln Ala Asp
           35                  40                  45
   Tyr Leu Glu Gln Asp Leu Ala Met Thr Lys Asp Gly Arg Leu Val Val
   50 55 60
   Ile His Asp His Phe Leu Asp Gly Leu Thr Asp Val Ala Lys Lys Phe
   65 70 75 80
   Pro His Arg His Arg Lys Asp Gly Arg Tyr Tyr Val Ile Asp Phe Thr
   85 90 95
   Leu Lys Glu Ile Gln Ser Leu Glu Met Thr Glu Asn Phe Glu Thr Met
   100 105 110
   Glu Arg Arg Arg Leu Trp Gly Ser Ile Gln Ser Arg Tyr Ile Ser Met
   115 120 125
   Ser Val Trp Thr Ser Pro Arg Arg Leu Val Glu Leu Ala Gly Gln Ser
   130 135 140
   Leu Leu Lys Asp Glu Ala Leu Ala Ile Ala Ala Leu Glu Leu Leu Pro
   145 150 155 160
   Arg Glu Leu Phe Pro Pro Leu Phe Met Ala Ala Phe Asp Gly Arg His
   165 170 175
   Ser Gln Thr Leu Lys Ala Met Val Gln Ala Trp Pro Phe Thr Cys Leu
   180 185 190
   Pro Leu Gly Val Leu Met Lys Gly Gln His Leu His Leu Glu Thr Phe
   195 200 205
   Lys Ala Val Leu Asp Gly Leu Asp Val Leu Leu Ala Gln Glu Val Arg
   210 215 220
   Pro Arg Arg Trp Lys Leu Gln Val Leu Asp Leu Arg Lys Asn Ser His
   225 230 235 240
   Gln Asp Phe Trp Thr Val Trp Ser Gly Asn Arg Ala Ser Leu Tyr Ser
   245 250 255
   Phe Pro Glu Pro Glu Ala Ala Gln Pro Met Thr Lys Lys Arg Lys Val
   260 265 270
   Asp Gly Leu Ser Thr Glu Ala Glu Gln Pro Phe Ile Pro Val Glu Val
   275 280 285
   Leu Val Asp Leu Phe Leu Lys Glu Gly Ala Cys Asp Glu Leu Phe Ser
   290 295 300
   Tyr Leu Ile Glu Lys Val Lys Arg Lys Lys Asn Val Leu Arg Leu Cys
   305 310 315 320
   Cys Lys Lys Leu Lys Ile Phe Ala Met Pro Met Gln Asp Ile Lys Met
   325 330 335
   Ile Leu Lys Met Val Gln Leu Asp Ser Ile Glu Asp Leu Glu Val Thr
   340 345 350
   Cys Thr Trp Lys Leu Pro Thr Leu Ala Lys Phe Ser Pro Tyr Leu Gly
   355 360 365
   Gln Met Ile Asn Leu Arg Arg Leu Leu Leu Ser His Ile His Ala Ser
   370 375 380
   Ser Tyr Ile Ser Pro Glu Lys Glu Glu Gln Tyr Ile Ala Gln Phe Thr
   385 390 395 400
   Ser Gln Phe Leu Ser Leu Gln Cys Leu Gln Ala Leu Tyr Val Asp Ser
   405 410 415
   Leu Phe Phe Leu Arg Gly Arg Leu Asp Gln Leu Leu Arg His Val Met
   420 425 430
   Asn Pro Leu Glu Thr Leu Ser Ile Thr Asn Cys Arg Leu Ser Glu Gly
   435 440 445
   Asp Val Met His Leu Ser Gln Ser Pro Ser Val Ser Gln Leu Ser Val
   450 455 460
   Leu Ser Leu Ser Gly Val Met Leu Thr Asp Val Ser Pro Glu Pro Leu
   465 470 475 480
   Gln Ala Leu Leu Glu Arg Ala Ser Ala Thr Leu Gln Asp Leu Val Phe
   485 490 495
   Asp Glu Cys Gly Ile Thr Asp Asp Gln Leu Leu Ala Leu Leu Pro Ser
   500 505 510
   Leu Ser His Cys Ser Gln Leu Thr Thr Leu Ser Phe Tyr Gly Asn Ser
   515 520 525
   Ile Ser Ile Ser Ala Leu Gln Ser Leu Leu Gln His Leu Ile Gly Leu
   530 535 540
   Ser Asn Leu Thr His Val Leu Tyr Pro Val Pro Leu Glu Ser Tyr Glu
   545 550 555 560
   Asp Ile His Gly Thr Leu His Leu Glu Arg Leu Ala Tyr Leu His Ala
   565 570 575
   Arg Leu Arg Glu Leu Leu Cys Glu Leu Gly Arg Pro Ser Met Val Trp
   580 585 590
   Leu Ser Ala Asn Pro Cys Pro His Cys Gly Asp Arg Thr Phe Tyr Asp
   595 600 605
   Pro Glu Pro Ile Leu Cys Pro Cys Phe Met Pro Asn His His His His
   610 615 620
   His His
   625
<210> 11
   <211> 1860
   <212> DNA
   <213> Artificial Sequence
<220>
   <223> MDP- 20-127 - Protein D - PRAME - no His tail (plasmid pET26)
<400> 11
   atggatccaa gcagccattc atcaaatatg gcgaataccc aaatgaaatc agacaaaatc 60
   attattgctc accgtggtgc tagcggttat ttaccagagc atacgttaga atctaaagca 120
   cttgcgtttg cacaacaggc tgattattta gagcaagatt tagcaatgac taaggatggt 180
   cgtttagtgg ttattcacga tcacttttta gatggcttga ctgatgttgc gaaaaaattc 240
   ccacatcgtc atcgtaaaga tggccgttac tatgtcatcg actttacctt aaaagaaatt 300
   caaagtttag aaatgacaga aaactttgaa accatggaac gtcgtcgtct gtggggcagc 360
   attcagagcc gttatattag catgagcgtg tggaccagcc cgcgtcgtct ggttgagctg 420
   gccggccaga gcctgctgaa agatgaagcg ctggccattg cggcgctgga gctgctgccg 480
   cgtgagctgt ttccgccgct gtttatggcg gcgtttgatg gccgtcatag ccagaccctg 540
   aaagcgatgg tgcaggcgtg gccgtttacc tgtctgccgc tgggcgtgct gatgaaaggc 600
   cagcatctgc atctggaaac ctttaaagcg gtgctggatg gcctggatgt gctgctggcc 660
   caggaagttc gtccgcgtcg ttggaaactg caagtgctgg atctgcgtaa aaacagccat 720
   caggattttt ggaccgtgtg gagcggcaat cgtgcgagcc tgtatagctt tccggaaccg 780
   gaagcggcgc agccgatgac caaaaaacgt aaagtggatg gcctgagcac cgaagcggaa 840
   cagccgttta ttccggtgga agtgctggtt gacctgtttc tgaaagaagg cgcctgcgac 900
   gagctgttta gctatctgat cgaaaaagtg aaacgcaaaa aaaacgtgct gcgtctgtgc 960
   tgcaaaaaac tgaaaatctt cgcgatgccg atgcaggata ttaaaatgat cctgaaaatg 1020
   gtgcagctgg atagcattga ggacctggaa gtgacctgca cctggaaact gccgaccctg 1080
   gccaaattta gcccgtatct gggccagatg attaacctgc gtcgtctgct gctgtctcat 1140
   attcatgcga gcagctatat tagcccggaa aaagaagaac agtatatcgc gcagtttacc 1200
   agccagtttc tgagcctgca atgcctgcaa gcgctgtatg tggatagcct gttttttctg 1260
   cgtggccgtc tggatcagct gctgcgtcat gtgatgaatc cgctggaaac cctgagcatt 1320
   accaactgcc gtctgagcga aggcgatgtg atgcatctga gccagagccc gagcgttagc 1380
   cagctgtctg ttctgagcct gagcggcgtg atgctgaccg atgtgagccc ggaaccgctg 1440
   caagccctgc tggaacgtgc gagcgcgacc ctgcaagacc tggtgtttga tgaatgcggc 1500
   attaccgatg atcagctgct ggccctgctg ccgagcctga gccattgcag ccagctgacc 1560
   accctgagct tttatggcaa cagcattagc attagcgcgc tgcaaagcct gctgcaacat 1620
   ctgattggcc tgagcaacct gacccatgtg ctgtatccgg tgccgctgga aagctatgaa 1680
   gatattcatg gcaccctgca tctggaacgt ctggcctatc tgcacgcgcg tctgcgtgag 1740
   ctgctgtgcg agctgggccg tccgagcatg gtttggctgt ctgcgaatcc gtgcccgcat 1800
   tgcggcgatc gtacctttta tgatccggaa ccgattctgt gcccgtgctt tatgccgaac 1860
<210> 12
   <211> 620
   <212> PRT
   <213> Artificial Sequence
<220>
   <223> MDP- 20-127 - Protein D - PRAME - no His tail (plasmid pET26)
<400> 12
   Met Asp Pro Ser Ser His Ser Ser Asn Met Ala Asn Thr Gln Met Lys
   1 5 10 15
   Ser Asp Lys Ile Ile Ile Ala His Arg Gly Ala Ser Gly Tyr Leu Pro
   20 25 30
   Glu His Thr Leu Glu Ser Lys Ala Leu Ala Phe Ala Gln Gln Ala Asp
   35 40 45
   Tyr Leu Glu Gln Asp Leu Ala Met Thr Lys Asp Gly Arg Leu Val Val
   50 55 60
   Ile His Asp His Phe Leu Asp Gly Leu Thr Asp Val Ala Lys Lys Phe
   65 70 75 80
   Pro His Arg His Arg Lys Asp Gly Arg Tyr Tyr Val Ile Asp Phe Thr
   85 90 95
   Leu Lys Glu Ile Gln Ser Leu Glu Met Thr Glu Asn Phe Glu Thr Met
   100 105 110
   Glu Arg Arg Arg Leu Trp Gly Ser Ile Gln Ser Arg Tyr Ile Ser Met
   115 120 125
   Ser Val Trp Thr Ser Pro Arg Arg Leu Val Glu Leu Ala Gly Gln Ser
   130 135 140
   Leu Leu Lys Asp Glu Ala Leu Ala Ile Ala Ala Leu Glu Leu Leu Pro
   145 150 155 160
   Arg Glu Leu Phe Pro Pro Leu Phe Met Ala Ala Phe Asp Gly Arg His
   165 170 175
   Ser Gln Thr Leu Lys Ala Met Val Gln Ala Trp Pro Phe Thr Cys Leu
   180 185 190
   Pro Leu Gly Val Leu Met Lys Gly Gln His Leu His Leu Glu Thr Phe
   195 200 205
   Lys Ala Val Leu Asp Gly Leu Asp Val Leu Leu Ala Gln Glu Val Arg
   210 215 220
   Pro Arg Arg Trp Lys Leu Gln Val Leu Asp Leu Arg Lys Asn Ser His
   225 230 235 240
   Gln Asp Phe Trp Thr Val Trp Ser Gly Asn Arg Ala Ser Leu Tyr Ser
   245 250 255
   Phe Pro Glu Pro Glu Ala Ala Gln Pro Met Thr Lys Lys Arg Lys Val
   260 265 270
   Asp Gly Leu Ser Thr Glu Ala Glu Gln Pro Phe Ile Pro Val Glu Val
   275 280 285
   Leu Val Asp Leu Phe Leu Lys Glu Gly Ala Cys Asp Glu Leu Phe Ser
   290 295 300
   Tyr Leu Ile Glu Lys Val Lys Arg Lys Lys Asn Val Leu Arg Leu Cys
   305 310 315 320
   Cys Lys Lys Leu Lys Ile Phe Ala Met Pro Met Gln Asp Ile Lys Met
   325 330 335
   Ile Leu Lys Met Val Gln Leu Asp Ser Ile Glu Asp Leu Glu Val Thr
   340 345 350
   Cys Thr Trp Lys Leu Pro Thr Leu Ala Lys Phe Ser Pro Tyr Leu Gly
   355 360 365
   Gln Met Ile Asn Leu Arg Arg Leu Leu Leu Ser His Ile His Ala Ser
   370 375 380
   Ser Tyr Ile Ser Pro Glu Lys Glu Glu Gln Tyr Ile Ala Gln Phe Thr
   385                 390                 395                 400
   Ser Gln Phe Leu Ser Leu Gln Cys Leu Gln Ala Leu Tyr Val Asp Ser
                   405                 410                 415
   Leu Phe Phe Leu Arg Gly Arg Leu Asp Gln Leu Leu Arg His Val Met
               420                 425                 430
   Asn Pro Leu Glu Thr Leu Ser Ile Thr Asn Cys Arg Leu Ser Glu Gly
           435                 440                 445
   Asp Val Met His Leu Ser Gln Ser Pro Ser Val Ser Gln Leu Ser Val
       450                 455                 460
   Leu Ser Leu Ser Gly Val Met Leu Thr Asp Val Ser Pro Glu Pro Leu
   465                 470                 475                 480
   Gln Ala Leu Leu Glu Arg Ala Ser Ala Thr Leu Gln Asp Leu Val Phe
                   485                 490                 495
   Asp Glu Cys Gly Ile Thr Asp Asp Gln Leu Leu Ala Leu Leu Pro Ser
               500                 505                 510
   Leu Ser His Cys Ser Gln Leu Thr Thr Leu Ser Phe Tyr Gly Asn Ser
           515                 520                 525
   Ile Ser Ile Ser Ala Leu Gln Ser Leu Leu Gln His Leu Ile Gly Leu
       530                 535                 540
   Ser Asn Leu Thr His Val Leu Tyr Pro Val Pro Leu Glu Ser Tyr Glu
   545                 550                 555                 560
   Asp Ile His Gly Thr Leu His Leu Glu Arg Leu Ala Tyr Leu His Ala
                   565                 570                 575
   Arg Leu Arg Glu Leu Leu Cys Glu Leu Gly Arg Pro Ser Met Val Trp
               580                 585                 590
   Leu Ser Ala Asn Pro Cys Pro His Cys Gly Asp Arg Thr Phe Tyr Asp
           595                 600                 605
   Pro Glu Pro Ile Leu Cys Pro Cys Phe Met Pro Asn
       610                 615                 620
<210> 13
   <211> 9
   <212> PRT
   <213> Homo sapiens
<400> 13
   Val Leu Asp Gly Leu Asp Val Leu Leu
   1               5
<210> 14
   <211> 10
   <212> PRT
   <213> Homo sapiens
<400> 14
   Ser Leu Tyr Ser Phe Pro Glu Pro Glu Ala
   1 5 10
<210> 15
   <211> 10
   <212> PRT
   <213> Homo sapiens
<400> 15
   Ala Leu Tyr Val Asp Ser Leu Phe Phe Leu
   1 5 10
<210> 16
   <211> 9
   <212> PRT
   <213> Homo sapiens
<400> 16
   Leu Tyr Val Asp Ser Leu Phe Phe Leu
   1 5
<210> 17
   <211> 9
   <212> PRT
   <213> Homo sapiens
<400> 17
   Ser Leu Leu Gln His Leu Ile Gly Leu
   1 5
<210> 18
   <211> 36
   <212> DNA
   <213> Artificial Sequence
<220>
   <223> sense oligonucleotide CAN008
<400> 18
   atataacata tggatccaag cagccattca tcaaat 36
<210> 19
   <211> 40
   <212> DNA
   <213> Artificial Sequence
<220>
   <223> antisense oligonucleotide CAN037
<400> 19
   ccacaaacgc cttcgttcca tggtttcaaa gttttctgtc 40
<210> 20
   <211> 40
   <212> DNA
   <213> Artificial Sequence
<220>
   <223> sense oligonucleotide CAN036
<400> 20
   gacagaaaac tttgaaacca tggaacgaag gcgtttgtgg 40
<210> 21
   <211> 39
   <212> DNA
   <213> Artificial Sequence
<220>
   <223> antisense oligonucleotide CAN029
<400> 21
   agagagacta gtctagttag gcatgaaaca ggggcacag 39
<210> 22
   <211> 36
   <212> DNA
   <213> Artificial Sequence
<220>
   <223> antisense oligonucleotide CAN002
<400> 22
   ggaggaacta gtgttaggca tgaaacaggg gcacag 36
<210> 23
   <211> 36
   <212> DNA
   <213> Artificial Sequence
<220>
   <223> sense oligonucleotide CAN040
<400> 23
   agagagcata tgagcagcca ttcatcaaat atggcg 36
<210> 24
   <211> 41
   <212> DNA
   <213> Artificial Sequence
<220>
   <223> antisense oligonucleotide CAN032
<400> 24
   acgtgggcgg ccgcggtttc aaagttttct gtcatttcta a 41
<210> 25
   <211> 39
   <212> DNA
   <213> Artificial Sequence
<220>
   <223> sense oligonucleotide CAN033
<400> 25
   ttgttggcgg ccgcaatgga acgaaggcgt ttgtggggt 39
<210> 26
   <211> 36
   <212> DNA
   <213> Artificial Sequence
<220>
   <223> antisense oligonucleotide CAN034
<400> 26
   ggaggactcg aggttaggca tgaaacaggg gcacag 36
<210> 27
   <211> 39
   <212> DNA
   <213> Artificial Sequence
<220>
   <223> antisense oligonucleotide CAN035
<400> 27
   ggaggactcg agctagttag gcatgaaaca ggggcacag 39
<210> 28
   <211> 31
   <212> DNA
   <213> Artificial Sequence
<220>
   <223> sense oligonucleotide CAN106
<400> 28
   cagaaaactt tgaaaccatg gaacgaaggc g 31
<210> 29
   <211> 31
   <212> DNA
   <213> Artificial Sequence
<220>
   <223> antisense oligonucleotide CAN107
<400> 29
   cgccttcgtt ccatggtttc aaagttttct g 31
<210> 30
   <211> 43
   <212> DNA
   <213> Artificial Sequence
<220>
   <223> sense oligonucleotide CAN104
<400> 30
   ggagatatac atatggatcc aagcagccat tcatcaaata tgg 43
<210> 31
   <211> 43
   <212> DNA
   <213> Artificial Sequence
<220>
   <223> antisense oligonucleotide CAN105
<400> 31
   ccatatttga tgaatggctg cttggatcca tatgtatatc tcc 43
<210> 32
   <211> 40
   <212> DNA
   <213> Artificial Sequence
<220>
   <223> sense oligonucleotide CAN123
<400> 32
   gacagaaaac tttgaaacca tggaacgtcg tcgtctgtgg 40
<210> 33
   <211> 40
   <212> DNA
   <213> Artificial Sequence
<220>
   <223> antisense oligonucleotide CAN124
<400> 33
   ccacagacga cgacgttcca tggtttcaaa gttttctgtc 40
<210> 34
   <211> 30
   <212> DNA
   <213> Artificial Sequence
<220>
   <223> sense oligonucleotide CAN199
<400> 34
   ggaattccat atggatccaa gcagccattc 30
<210> 35
   <211> 53
   <212> DNA
   <213> Artificial Sequence
<220>
   <223> antisense oligonucleotide CAN19
<400> 35
   ggagctctcg agtcagtggt ggtggtggtg gtggttcggc ataaagcacg ggc 53
<210> 36
   <211> 20
   <212> DNA
   <213> Artificial Sequence
<220>
   <223> immunostimulatory oligonucleotide CpG 1826
<400> 36
   tccatgacgt tcctgacgtt 20
<210> 37
   <211> 18
   <212> DNA
   <213> Artificial Sequence
<220>
   <223> immunostimulatory oligonucleotide CpG 1758
<400> 37
   tctcccagcg tgcgccat 18
<210> 38
   <211> 54
   <212> DNA
   <213> Artificial Sequence
<220>
   <223> immunostimulatory oligonucleotide CpG2006
<400> 38
   accgatgacg tcgccggtga cggcaccacg tcgtcgtttt gtcgttttgt cgtt 54
<210> 39
   <211> 20
   <212> DNA
   <213> Artificial Sequence
<220>
   <223> immunostimulatory oligonucleotide CpG1668
<400> 39
   tccatgacgt tcctgatgct 20
<210> 40
   <211> 22
   <212> DNA
   <213> Artificial Sequence
<220>
   <223> immunostimulatory oligonucleotide CpG5456
<400> 40
   tcgacgtttt cggcgcgcgc cg 22
<210> 41
   <211> 127
   <212> PRT
   <213> Haemophilus influenzae b
<400> 41
   Met Lys Leu Lys Thr Leu Ala Leu Ser Leu Leu Ala Ala Gly Val Leu
    1               5                  10                  15
   Ala Gly Cys Ser Ser His Ser Ser Asn Met Ala Asn Thr Gln Met Lys
               20                  25                  30
   Ser Asp Lys Ile Ile Ile Ala His Arg Gly Ala Ser Gly Tyr Leu Pro
           35                  40                  45
   Glu His Thr Leu Glu Ser Lys Ala Leu Ala Phe Ala Gln Gln Ala Asp
       50                  55                  60
   Tyr Leu Glu Gln Asp Leu Ala Met Thr Lys Asp Gly Arg Leu Val Val
   65                  70                  75                  80
   Ile His Asp His Phe Leu Asp Gly Leu Thr Asp Val Ala Lys Lys Phe
                   85                  90                  95
   Pro His Arg His Arg Lys Asp Gly Arg Tyr Tyr Val Ile Asp Phe Thr
               100                 105                 110
   Leu Lys Glu Ile Gln Ser Leu Glu Met Thr Glu Asn Phe Glu Thr
           115                 120                 125
<210> 42
   <211> 111
   <212> PRT
   <213> Artificial Sequence
<220>
   <223> 1-Met; 2-Asp; 3-Pro; followed by AA 20 to 127 of Protein D
<400> 42
   Met Asp Pro Ser Ser His Ser Ser Asn Met Ala Asn Thr Gln Met Lys
   1 5 10 15
   Ser Asp Lys Ile Ile Ile Ala His Arg Gly Ala Ser Gly Tyr Leu Pro
   20 25 30
   Glu His Thr Leu Glu Ser Lys Ala Leu Ala Phe Ala Gln Gln Ala Asp
   35 40 45
   Tyr Leu Glu Gln Asp Leu Ala Met Thr Lys Asp Gly Arg Leu Val Val
   50 55 60
   Ile His Asp His Phe Leu Asp Gly Leu Thr Asp Val Ala Lys Lys Phe
   65 70 75 80
   Pro His Arg His Arg Lys Asp Gly Arg Tyr Tyr Val Ile Asp Phe Thr
   85 90 95
   Leu Lys Glu Ile Gln Ser Leu Glu Met Thr Glu Asn Phe Glu Thr
   100 105 110
<210> 43
   <211> 450
   <212> PRT
   <213> Artificial Sequence
<220>
   <223> Protein D-MAGE-A3-His
<400> 43
   Met Asp Pro Lys Thr Leu Ala Leu Ser Leu Leu Ala Ala Gly Val Leu
   1 5 10 15
   Ala Gly Cys Ser Ser His Ser Ser Asn Met Ala Asn Thr Gln Met Lys
   20 25 30
   Ser Asp Lys Ile Ile Ile Ala His Arg Gly Ala Ser Gly Tyr Leu Pro
   35 40 45
   Glu His Thr Leu Glu Ser Lys Ala Leu Ala Phe Ala Gln Gln Ala Asp
   50 55 60
   Tyr Leu Glu Gln Asp Leu Ala Met Thr Lys Asp Gly Arg Leu Val Val
   65 70 75 80
   Ile His Asp His Phe Leu Asp Gly Leu Thr Asp Val Ala Lys Lys Phe
   85 90 95
   Pro His Arg His Arg Lys Asp Gly Arg Tyr Tyr Val Ile Asp Phe Thr
   100 105 110
   Leu Lys Glu Ile Gln Ser Leu Glu Met Thr Glu Asn Phe Glu Thr Met
   115 120 125
   Asp Leu Glu Gln Arg Ser Gln His Cys Lys Pro Glu Glu Gly Leu Glu
   130 135 140
   Ala Arg Gly Glu Ala Leu Gly Leu Val Gly Ala Gln Ala Pro Ala Thr
   145 150 155 160
   Glu Glu Gln Glu Ala Ala Ser Ser Ser Ser Thr Leu Val Glu Val Thr
   165 170 175
   Leu Gly Glu Val Pro Ala Ala Glu Ser Pro Asp Pro Pro Gln Ser Pro
   180 185 190
   Gln Gly Ala Ser Ser Leu Pro Thr Thr Met Asn Tyr Pro Leu Trp Ser
   195 200 205
   Gln Ser Tyr Glu Asp Ser Ser Asn Gln Glu Glu Glu Gly Pro Ser Thr
   210 215 220
   Phe Pro Asp Leu Glu Ser Glu Phe Gln Ala Ala Leu Ser Arg Lys Val
   225 230 235 240
   Ala Glu Leu Val His Phe Leu Leu Leu Lys Tyr Arg Ala Arg Glu Pro
   245 250 255
   Val Thr Lys Ala Glu Met Leu Gly Ser Val Val Gly Asn Trp Gln Tyr
   260 265 270
   Phe Phe Pro Val Ile Phe Ser Lys Ala Ser Ser Ser Leu Gln Leu Val
   275 280 285
   Phe Gly Ile Glu Leu Met Glu Val Asp Pro Ile Gly His Leu Tyr Ile
   290 295 300
   Phe Ala Thr Cys Leu Gly Leu Ser Tyr Asp Gly Leu Leu Gly Asp Asn
   305 310 315 320
   Gln Ile Met Pro Lys Ala Gly Leu Leu Ile Ile Val Leu Ala Ile Ile
   325 330 335
   Ala Arg Glu Gly Asp Cys Ala Pro Glu Glu Lys Ile Trp Glu Glu Leu
   340 345 350
   Ser Val Leu Glu Val Phe Glu Gly Arg Glu Asp Ser Ile Leu Gly Asp
   355 360 365
   Pro Lys Lys Leu Leu Thr Gln His Phe Val Gln Glu Asn Tyr Leu Glu
   370 375 380
   Tyr Arg Gln Val Pro Gly Ser Asp Pro Ala Cys Tyr Glu Phe Leu Trp
   385 390 395 400
   Gly Pro Arg Ala Leu Val Glu Thr Ser Tyr Val Lys Val Leu His His
   405 410 415
   Met Val Lys Ile Ser Gly Gly Pro His Ile Ser Tyr Pro Pro Leu His
   420 425 430
   Glu Trp Val Leu Arg Glu Gly Glu Glu Gly Gly His His His His His
   435 440 445
   His His
   450
<210> 44
   <211> 109
   <212> PRT
   <213> Artificial Sequence
<220>
   <223> the aa from protein D from Haemophilus influenzae for use in a pDl/3-PRAME-His sequence
<400> 44
   Met Ser Ser His Ser Ser Asn Met Ala Asn Thr Gln Met Lys Ser Asp
   1 5 10 15
   Lys Ile Ile Ile Ala His Arg Gly Ala Ser Gly Tyr Leu Pro Glu His
   20 25 30
   Thr Leu Glu Ser Lys Ala Leu Ala Phe Ala Gln Gln Ala Asp Tyr Leu
   35 40 45
   Glu Gln Asp Leu Ala Met Thr Lys Asp Gly Arg Leu Val Val Ile His
   50 55 60
   Asp His Phe Leu Asp Gly Leu Thr Asp Val Ala Lys Lys Phe Pro His
   65 70 75 80
   Arg His Arg Lys Asp Gly Arg Tyr Tyr Val Ile Asp Phe Thr Leu Lys
   85 90 95
   Glu Ile Gln Ser Leu Glu Met Thr Glu Asn Phe Glu Thr
   100 105
<210> 45
   <211> 127
   <212> PRT
   <213> Artificial Sequence
<220>
   <223> the aa from protein D from Haemophilus influenzae for use in a pDl/3-MAGE-His sequence
<400> 45
   Met Asp Pro Lys Thr Leu Ala Leu Ser Leu Leu Ala Ala Gly Val Leu
    1              5                   10                  15
   Ala Gly Cys Ser Ser His Ser Ser Asn Met Ala Asn Thr Gln Met Lys
               20                  25                  30
   Ser Asp Lys Ile Ile Ile Ala His Arg Gly Ala Ser Gly Tyr Leu Pro
           35                  40                  45
   Glu His Thr Leu Glu Ser Lys Ala Leu Ala Phe Ala Gln Gln Ala Asp
       50                  55                  60
   Tyr Leu Glu Gln Asp Leu Ala Met Thr Lys Asp Gly Arg Leu Val Val
   65                  70                  75                  80
   Ile His Asp His Phe Leu Asp Gly Leu Thr Asp Val Ala Lys Lys Phe
                   85                  90                  95
   Pro His Arg His Arg Lys Asp Gly Arg Tyr Tyr Val Ile Asp Phe Thr
               100                 105                 110
   Leu Lys Glu Ile Gln Ser Leu Glu Met Thr Glu Asn Phe Glu Thr
           115                 120                 125

## Description

The present invention relates to a fusion protein comprising an antigen derived from the so-called tumour rejection antigen PRAME (also known as DAGE) linked to an immunological fusion partner derived from protein D from *Haemophilus influenzae* B, methods for preparing the same and for formulating vaccines and use of the same for treating a range of cancers, including, but not limited to melanoma, breast, bladder, lung cancer such as NSCLC, sarcoma, ovarian cancer, head and neck cancer, renal cancer, colorectal carcinoma, multiple myeloma, leukemia including acute leukemia and oesophageal carcinoma.

Among different groups of tumour-associated antigens, cancer testis antigens are of interest for immunotherapy because of their broad tumour-specific expression and the fact that generally these antigens are not expressed in healthy cells. More than 50 cancer/testis antigens have been described so far and, for many of them, epitopes recognized by T lymphocytes have been identified. PRAME is a cancer testis antigen and is in under investigation as a potential immunotherapy.

In immunotherapy the cancer antigen is introduced to the patient usually as a vaccine, for example containing an antigen as a protein or an immunogenic fragment thereof, or as DNA encoding for the protein or as a vector containing said DNA, which stimulates the patient's immune system to attack tumours expressing the same antigen.

If the appropriate response is stimulated, T lymphocytes (T cells) attack antigens directly, and provide control of the immune response. B cells and T cells develop that are specific for one antigen type. When the immune system is exposed to a different antigen, different B cells and T cells are formed. As lymphocytes develop, they normally learn to recognize the body's own tissues (self) as different from tissues and particles not normally found in the body (non-self). Once B cells and T cells are formed, a few of those cells will multiply and provide "memory" for the immune system. This allows the immune system to respond faster and more efficiently the next time it is exposed to the same antigen.
Certain experiments seem to indicate that cancer testis antigens can stimulate the memory mechanisms in the immune system.

It is hypothesized by some that PRAME is involved in cell death or cell cycles. It has been shown by some groups to be expressed in melanoma and a wide variety of tumours including lung, kidney and head and neck. Interestingly it also seems to be expressed in 40-60% leukemia such as acute lymphoid leukemia and acute myeloid leukemia, see for example Exp Hematol. 2000 Dec;28(12):1413-22. In patients it has been observed that over expression ofPRAME seems to be associated with higher survival and lower rates of relapse in comparison to those who do not over express the protein.

The antigen and its preparation are described in US patent No. 5, 830, 753. PRAME is found in the Annotated Human Gene Database H-Inv DB under the accession numbers: U65011.1, BC022008.1, AK129783.1, BC014974.2, CR608334.1, AF025440.1, CR591755.1, BC039731.1, CR623010.1, CR611321.1, CR618501.1, CR604772.1, CR456549.1, and CR620272.1.

Protein D is a surface protein of the gram-negative bacterium, Haemophilus influenza B. Information on immunological fusion partners derived from protein D can be obtained from WO 91/18926.

Fusion proteins of a portion of an antigen and a heterologous fusion partner are sometimes prepared to increase the immunogenicity of the antigen and/or aid production of the protein in appropriate quantities and/or purity see for example WO 99/40188 which describes a fusion protein of MAGE and, for example protein D a surface protein of the gram-negative bacterium, *Haemophilus influenza* B. The fusion protein is prepared recombinantly and the protein D secretion sequence can be incorporated into the fusion protein to potentially assist secretion and solubilisation of the final product.

WO00/50077 describes a number of peptide immunogens linked to a carrier wherein the carrier is derived from Haemophilius Influenzae Protein D or fragments thereof. WO99/10375 describes Human Papilloma Virus (HPV) fusion proteins, linked to an immunological fusion partner that provides T helper epiptopes to the HPV antigen.

### Brief Description of the Figures and Constructs/Sequences

| | |
|---|---|
| Figure 1 | SDS-page analysis of Construct 3 and 4 |
| Figure 2 | SDS-page analysis of Construct 3a and 4a |
| Figure 3 | CD4 response (AS01B adjuvant) |
| Figure 4 | CD8 response (AS01B adjuvant - 20070499) |
| Figure 5 | CD4 response (AS15 adjuvant) |
| Figure 6 | CD8 response (AS 15 adjuvant) |
| Figure 7 | A marked up amino acid sequence of examples of constructs of the present invention |
| Figure 8 | Alignment between LipoD-MAGE3-His (SEQ ID NO:43) and pD1/3-PRAME-His (SEQ ID NO:10) |
| Figure 9 | Alignment between the shared sequence of the original protein D from *Haemophilus influenzae* (SEQ ID NO:45) and the LipoD-MAGE3-His (SEQ ID NO:43) |
| Figure 10 | Alignment between the shared sequence of the original protein D from *Haemophilus influenzae* (SEQ ID NO:41), the pD-MAGE3-His (SEQ ID NO:45) and a construct of pD1/3-PRAME-His which does not include the amino acids 2-D and 3-P (SEQ ID NO:44) |
| Figure 11 | SDS page analysis of pD1/3-PRAME with or without secretion signal (SS) and His-tail (His) in pET21 vector. |
| Figure 12 | Sequence of protein D 1/3 MAGE-A3-His (SEQ ID NO:44) |
| Construct 1 (Example 1) | MDP- 20-127 - Protein D - PRAME - TSGHHHHHH (plasmid TCMP14) (nucleotide sequence SEQ ID NO: 1; amino acid sequence SEQ ID NO:2) |
| Construct 2 (Example 2) | MDP- 20-127 - Protein D - PRAME - no His tail (plasmid TCMP 14) (nucleotide sequence SEQ ID NO: 3; amino acid sequence SEQ ID NO:4) |
| Construct 3 (Example 3) | MDP- 20-127 - Protein D - PRAME - LEHHHHHH (plasmid pET21) (nucleotide sequence SEQ ID NO: 5; amino acid sequence SEQ ID NO:6) |
| Construct 4 (Example 4) | MDP- 20-127 - Protein D - PRAME - no His tail (plasmid pET21) (nucleotide sequence SEQ ID NO: 7; amino acid sequence SEQ ID NO:8) |
| Construct 3a (Example 3a) | MDP- 20-127 - Protein D - PRAME - HHHHHH (plasmid pET26) (nucleotide sequence SEQ ID NO: 9; amino acid sequence SEQ ID NO:10) |
| Construct 4a (Example 4a) | MDP- 20-127 - Protein D - PRAME - no His tail (plasmid pET26) (nucleotide sequence SEQ ID NO: 11; amino acid sequence SEQ ID NO:12) |
| Sequence 1 (SEQ ID NO:1) | is the DNA sequence for Example 1 |
| Sequence 2 (SEQ ID NO:2) | is the amino acid sequence for Example 1 |
| Sequence 3 (SEQ ID NO:3) | is the DNA sequence for Example 2 |
| Sequence 4 (SEQ ID NO:4) | is the amino acid sequence for Example 2 |
| Sequence 5 (SEQ ID NO:5) | is the DNA sequence for Example 3 |
| Sequence 6 (SEQ ID NO:6) | is the amino acid sequence for Example 3 |
| Sequence 7 (SEQ ID NO:7) | is the DNA sequence for Example 4 |
| Sequence 8 (SEQ ID NO:8) | is the amino acid sequence for Example4 |
| Sequence 9 (SEQ ID NO:9) | is the codon optimized DNA sequence for Example 3a |
| Sequence 10 (SEQ ID NO:10) | is the amino acid sequence for Example 3a |
| Sequence 11 (SEQ ID NO:11) | is the codon optimized DNA sequence for Example 4a |
| Sequence 12 (SEQ ID NO:12) | is the amino acid sequence for Example 4a |
| SEQ ID NO:13 | VLDGLDVLL (PRA¹⁰⁰⁻¹⁰⁸) |
| SEQ ID NO:14 | SLYSFPEPEA (PRA¹⁴²⁻¹⁵¹) |
| SEQ ID NO:15 | ALYVDSLFFL (PRA³⁰⁰⁻³⁰⁹) |
| SEQ ID NO:16 | LYVDSLFFL (PRA³⁰¹⁻³⁰⁹) |
| SEQ ID NO:17 | SLLQHLIGL (PRA⁴²⁵⁻⁴³³) |
| SEQ ID NO:18 to 35 | Oligonucleotides used in the examples of the present invention |
| SEQ ID NO: 36 | TCC ATG ACG TTC CTG ACG TT (CpG 1826;) |
| SEQ ID NO: 37 | TCT CCC AGC GTG CGC CAT (CpG 1758) |
| SEQ ID NO: 38 | ACC GAT GAC GTC GCC GGT GAC GGC ACC ACG TCG TCG TTT TGT CGT TTT GTC GTT (CpG 2006) |
| SEQ ID NO: 39 | TCC ATG ACG TTC CTG ATG CT (CpG 1668) |
| SEQ ID NO: 40 | TCG ACG TTT TCG GCG CGC GCC G (CpG 5456) |
| SEQ ID NO:41 | AA 1 to 127 of Protein D from *Haemophilus influenzae* |
| SEQ ID NO:42 | 1-Met; 2-Asp; 3-Pro; followed by AA 20 to 127 of Protein D |
| SEQ ID NO:43 | Amino acid sequence of protein D 1/3- MAGE-A3 -His |
| SEQ ID NO:44 | Amino acids from protein D from Haemophilus influenzae for use in a pD1/3- |

| | PRAME-His sequence |
|---|---|
| SEQ ID NO:45 | Amino acids from protein D from Haemophilus influenzae for use in a pD1/3-MAGE-His sequence |

### Summary of the invention

The present invention provides a fusion protein comprising:
a) PRAME;
b) a heterologous fusion partner derived from protein D,
wherein the fusion protein comprises amino acids 20 to 127 of protein D; and c) additional amino acids Met-Asp-Pro at the N-terminal end of the fusion protein sequence wherein the heterologous fusion partner protein derived from protein O (b), is fused to the N-terminus of PRAME

It seems that for fusion proteins comprising PRAME or an immunogenic fragment thereof and protein D, or for fusion proteins comprising protein D, or for a fusion partner protein comprising protein D, that the presence of the secretion sequence (or signal sequence) may detrimentally affect the amount of fusion protein produced.

### PRAME

In one aspect the fusion protein of the present invention comprises a fusion partner protein as described herein and a PRAME antigen or immunogenic fragment thereof. Generally the PRAME protein has 509 amino acids and in one embodiment all 509 amino acids of PRAME may be used. Several cytotoxic T lymphocytle (CTL) epitopes have been identified on PRAME, for example:
VLDGLDVLL (PRA¹⁰⁰⁻¹⁰⁸; SEQ ID NO:13);
SLYSFPEPEA (PRA¹⁴²⁻¹⁵¹; SEQ ID NO:14);
ALYVDSLFFL (PRA³⁰⁰⁻³⁰⁹; SEQ ID NO:15);
LYVDSLFFL (PRA³⁰¹⁻³⁰⁹; SEQ ID NO: 16) and
SLLQHLIGL (PRA⁴²⁵⁻⁴³³; SEQ ID NO:17).

Generally it is desirable to include as many of these epitopes as possible into the antigen to generate a strong immune response and ensure the antigen is as immunogenic as possible. Although, it may be possible to compensate for a lower immunogenicity of a given construct by employing a formulation with a potent immunological adjuvant. Strong adjuvants are discussed below in more detail.

In one aspect the invention provides the PRAME portion of the fusion protein comprising, consisting of or consisting essentially of full length protein.

However, the invention also extends to PRAME constructs with conservative substitutions. In one embodiment, 1, 2, 3, 4, 5, 6, 7, 8, 9 or more amino acids may be substituted. The PRAME construct as described herein may additionally or alternatively contain deletions or insertions within the amino acid sequence when compared to the wildtype PRAME sequence. In one embodiment, 1, 2, 3, 4, 5, 6, 7, 8, 9 or more amino acids may be inserted or deleted.

Conservative substitutions are well known and are generally set up as the default scoring matrices in sequence alignment computer programs. These programs include PAM250 (Dayhoft M.O. et al., (1978), "A model of evolutionary changes in proteins", In "Atlas of Protein sequence and structure" 5(3) M.O. Dayhoft (ed.), 345-352), National Biomedical Research Foundation, Washington, and Blosum 62 (Steven Henikoft and Jorja G. Henikoft (1992), "Amino acid substitution matricies from protein blocks"), Proc. Natl. Acad. Sci. USA 89 (Biochemistry): 10915-10919.

In general terms, substitution within the following groups are conservative substitutions, but substitutions between groups are considered non-conserved. The groups are:
i) Aspartate/asparagine/glutamate/glutamine
ii) Serine/threonine
iii) Lysine/arginine
iv) Phenylalanine/tyrosine/tryptophane
v) Leucine/isoleucine/valine/methionine
vi) Glycine/alanine

Generally the PRAME sequence/amino acids used in the fusion proteins of the invention will be greater than 80%, such as 85, 90, 95 and more specifically 99% identical to naturally occurring PRAME. However, those skilled in the art are aware that amino acid residues generated as a result of the cloning process may be retained in the recombinantly synthesized proteins. If these do not detrimentally affect the characteristics of the product, it is optional whether or not they are removed.

In one aspect the invention provides a fusion protein as described herein comprising, consisting of or consisting essentially of full length PRAME protein. In a further aspect the PRAME portion of the fusion protein of the present invention comprises, consists of or consists essentially of one or more of the following epitopes:
VLDGLDVLL (PRA¹⁰⁰⁻¹⁰⁸; SEQ ID NO:13);
SLYSFPEPEA (PRA¹⁴²⁻¹⁵¹; SEQ ID NO:14);
ALYVDSLFFL (PRA³⁰⁰⁻³⁰⁹; SEQ ID NO:15);
LYVDSLFFL (PRA³⁰¹⁻³⁰⁹; SEQ ID NO:16) and
SLLQHLIGL (PRA⁴²⁵⁻⁴³³; SEQ ID NO:17).

The fusion proteins of the PRAME antigen and protein D fusion partner protein as described herein are preferably expressed as recombinant fusion proteins, which may allow increased levels of PRAME protein to be produced in an expression system as compared to PRAME alone without fusion partner, such as protein D or modified protein D proteins.

Fusion proteins of the present invention, as described herein, may additionally comprise one or more linker sequences between the fusion partner protein and the tumour antigen or immunogenic portion thereof; or between the fusion partner protein and a His tail or other affinity tag (if present); or between the tumour antigen or immunogenic portion thereof and a His tail or other affinity tag(if present). The amino acids in the linker sequences may be unrelated to the sequences of the antigen and/or fusion partner.

The fusion partner may assist in expressing the protein (expression enhancer) at higher yields than the native recombinant protein. The fusion partner protein D, due to its foreign nature, may be particularly immunogenic *in vivo* and assist the fusion protein comprising PRAME by providing T helper epitopes, preferably T helper epitopes recognised by CD4 T-cells. Such CD4-T cells may be believed to contribute to generating a favourable immune response, in particular, a CD8 cytolytic T-cell response.
In one embodiment, the fusion partner may act as both an expression enhancing partner and an immunological fusion partner.

In one aspect the invention provides a fusion protein wherein the N-terminal portion of protein D (as described above or herein) is fused to the N-terminus of PRAME . More specifically the fusion with the protein D fragment and the N-terminus of PRAME is effected such that the PRAME replaces the C-terminal-fragment of protein D that has been excised. Thus the N-terminus of protein D becomes the N-terminus of the fusion protein.

Fusion proteins of the invention may include an affinity tag, such as for example, a histidine tail comprising between 5 to 9 such as 6 histidine residues. These residues may, for example be on the terminal portion of protein D (such as the N-terminal of protein D) and/or the may be fused to the terminal portion of the PRAME antigen or derivative thereof, or the tumour antigen or derivative thereof as described herein. Generally however the histidine tail with be located on terminal portion of the PRAME antigen or derivative thereof, or the tumour antigen or derivative thereof as described herein such as the C-terminal end of the PRAME antigen or derivative thereof, or the tumour antigen or derivative thereof as described herein. Histidine tails may be advantageous in aiding purification.

The present invention also provides a nucleic acid encoding the proteins of the present invention. Such sequences can be inserted into a suitable expression vector and used for DNA/RNA vaccination or expressed in a suitable host. Microbial vectors expressing the nucleic acid may be used as vaccines. Such vectors include for example, poxvirus, adenovirus, alphavirus, listeria and monophage.

A DNA sequence encoding the proteins of the present invention can be synthesized using standard DNA synthesis techniques, such as by enzymatic ligation as described by D.M. Roberts et al. in Biochemistry 1985, 24, 5090-5098, by chemical synthesis, by *in vitro* enzymatic polymerization, or by PCR technology utilising for example a heat stable polymerase, or by a combination of these techniques.

Enzymatic polymerisation of DNA may be carried out *in vitro* using a DNA polymerase such as DNA polymerase I (Klenow fragment) in an appropriate bluffer containing the nucleoside triphosphates dATP, dCTP, dGTP and dTTP as required at a temperature of 10°-37°C, generally in a volume of 50µl or less. Enzymatic ligation of DNA fragments may be carried out using a DNA ligase such as T4 DNA ligase in an appropriate buffer, such as 0.05M Tris (pH 7.4), 0.01M MgCl₂, 0.01M dithiothreitol, 1mM spermidine, 1mM ATP and 0.1mg/ml bovine serum albumin, at a temperature of 4°C to ambient, generally in a volume of 50ml or less. The chemical synthesis of the DNA polymer or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982), or in other scientific publications, for example M.J. Gait, H.W.D. Matthes, M. Singh, B.S. Sproat, and R.C. Titmas, Nucleic Acids Research, 1982, 10, 6243; B.S. Sproat, and W. Bannwarth, Tetrahedron Letters, 1983, 24, 5771; M.D. Matteucci and M.H. Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society, 1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801.

The process of the invention may be performed by conventional recombinant techniques such as described in Maniatis et al., Molecular Cloning - A Laboratory Manual; Cold Spring Harbor, 1982-1989.

In particular, the process may comprise the steps of :
i) preparing a replicable or integrating expression vector capable, in a host cell, of expressing a DNA polymer comprising a nucleotide sequence that encodes the protein or an immunogenic derivative thereof;
ii) transforming a host cell with said vector;
iii) culturing said transformed host cell under conditions permitting expression of said DNA polymer to produce said protein; and
iv) recovering said protein.
The term 'transforming' is used herein to mean the introduction of foreign DNA into a host cell. This can be achieved for example by transformation, transfection or infection with an appropriate plasmid or viral vector using e.g. conventional techniques as described in Genetic Engineering; Eds. S.M. Kingsman and A.J. Kingsman; Blackwell Scientific Publications; Oxford, England, 1988. The term `transformed' or 'transformant' will hereafter apply to the resulting host cell containing and expressing the foreign gene of interest.

The expression vectors are novel and also form part of the invention.

The replicable expression vectors may be prepared in accordance with the invention, by cleaving a vector compatible with the host cell to provide a linear DNA segment having an intact replicon, and combining said linear segment with one or more DNA molecules which, together with said linear segment encode the desired product, such as the DNA polymer encoding the protein of the invention, or derivative thereof, under ligating conditions.

Thus, the DNA polymer may be preformed or formed during the construction of the vector, as desired.

The choice of vector will be determined in part by the host cell, which may be prokaryotic or eukaryotic but are generally *E. coli* or CHO cells. Suitable vectors may include plasmids for example TMCP14 or pET21 or pET26, pcDNA3, bacteriophages, cosmids and recombinant viruses.

The preparation of the replicable expression vector may be carried out conventionally with appropriate enzymes for restriction, polymerisation and ligation of the DNA, by procedures described in, for example, Maniatis *et al.* cited above.

The recombinant host cell is prepared, in accordance with the invention, by transforming a host cell with a replicable expression vector of the invention under transforming conditions. Suitable transforming conditions are conventional and are described in, for example, Maniatis *et al.* cited above, or "DNA Cloning" Vol. II, D.M. Glover ed., IRL Press Ltd, 1985.

The choice of transforming conditions is determined by the host cell. Thus, a bacterial host such as E. *coli* may be treated with a solution of CaCl₂ (Cohen et al., Proc. Nat. Acad. Sci., 1973, 69, 2110) or with a solution comprising a mixture of RbCl, MnCl₂, potassium acetate and glycerol, and then with 3-[N-morpholino]-propane-sulphonic acid, RbCl and glycerol. Mammalian cells in culture may be transformed by calcium coprecipitation of the vector DNA onto the cells. The invention also extends to a host cell transformed with a replicable expression vector of the invention.

The DNA may be codon optimized by standard techniques to further facilitate expression of the relevant host. In one embodiment of the present invention there is provided DNA encoding a fusion protein comprising a FRAME antigen or portion or fragment thereof as described herein, in which the nucleotide sequence of the PRAME antigen or portion or fragment thereof is codon-optimised. In one embodiment, the protein D nucleotide sequence is not codon-optimised.

Culturing the transformed host cell under conditions permitting expression of the DNA polymer is carried out conventionally, as described in, for example, Maniatis *et al.* and "DNA Cloning" cited above. Thus, preferably the cell is supplied with nutrient and cultured at a temperature below 50°C.

The proteins of the present invention may be expressed in prokaryotes or eukaryotes such as yeast but are often expressed in *E. coli.* Particular strains of *E. coli* such as AR58 and BLR DE3 may be employed.

Generally a selection marker of, for example kanamycine resistance or ampicillin resistance is incorporated to facilitate identification of the successful incorporation of the recombinant gene/construct into the expression system.

The product is recovered by conventional methods according to the host cell and according to the localisation of the expression product (intracellular or secreted into the culture medium or into the cell periplasm). In one embodiment of the present invention the expression product is intracellular. In one embodiment of the present invention the expression product is an insoluble protein. Thus, where the host cell is bacterial, such as *E. coli* it may, for example, be lysed physically, chemically or enzymatically and the protein product isolated from the resulting lysate. Where the host cell is mammalian, the product may generally be isolated from the nutrient medium or from cell free extracts. Conventional protein isolation techniques include selective precipitation, adsorption chromatography, and affinity chromatography including a monoclonal antibody affinity column.

In one embodiment of the invention there is provided a process for producing a fusion protein as described herein comprising the step of expressing in a cell a fusion protein comprising a fusion partner protein as described herein. The cell may be a bacterium. In one embodiment in which the cell is a bacterium, the bacterium may be *E. coli.* The process of the present invention may comprise the step of expressing a fusion protein as described herein in a cell as an insoluble protein. The process may further comprise the step of lysing the cell and purifying the expressed fusion protein from the lysed cells.
In one embodiment of the invention there is provided a fusion protein obtained by or obtainable by a method or process described herein.

The proteins of the present invention are provided either soluble in a liquid form or in a lyophilised form.

It is generally expected that each human dose will comprise 1 to 1000 µg of protein, and preferably 30 - 300 µg.

The present invention also provides pharmaceutical composition such as vaccine comprising a fusion protein of the present invention in a pharmaceutically acceptable excipient.

The vaccine may optionally contain one or more other tumour-associated antigens or polypeptides, or preferably be combined with other cancer vaccines based on a tumour-associated antigen. For example, these tumour-associated antigens could be antigens as described herein and/or may be members belonging to the MAGE, LAGE and GAGE families or WT-1. In one embodiment the tumour-associated antigen may comprise or consist of the MAGE-A3 antigen.

Vaccine preparation is generally described in Vaccine Design ("The subunit and adjuvant approach" (eds. Powell M.F. & Newman M.J). (1995) Plenum Press New York). Encapsulation within liposomes is described by Fullerton, US Patent 4,235,877.

The proteins of the present invention may be preferably adjuvanted in the vaccine formulation of the invention. Suitable adjuvants may include an aluminium salt such as aluminium hydroxide gel (alum) or aluminium phosphate, but may also be a salt of calcium, iron or zinc, or may be an insoluble suspension of acylated tyrosine, or acylated sugars, cationically or anionically derivatised polysaccharides, or polyphosphazenes. Other known adjuvants include CpG containing oligonucleotides. The oligonucleotides are characterised in that the CpG dinucleotide is unmethylated. Such oligonucleotides are well known and are described in, for example WO 96/02555.

In the formulation of the inventions it may be desirable that the adjuvant composition induces an immune response preferentially of the TH1 type. In one embodiment there is provided an adjuvant system including, for example a combination of monophosphoryl lipid A, preferably 3-de-O-acylated monophosphoryl lipid A (3D-MPL) together with an aluminium salt. The adjuvant may optionally also include CpG oligonucleotides to preferentially induce a TH1 response.

An enhanced system that may be used in the present invention comprises the combination of a monophosphoryl lipid A and a saponin derivative particularly the combination of QS21 and 3D-MPL as disclosed in WO 94/00153, or, for example a less reactogenic composition where the QS21 is quenched with cholesterol as disclosed in WO 96/33739.

A formulation that may be used in formulations of the present invention, comprising QS21 3D-MPL & tocopherol, for example in an oil in water emulsion, is described in WO 95/17210.
Another adjuvant formulation that may be used in formulations of the present invention is QS21, 3D-MPL & CpG or equivalent thereof, for example in an oil in water emulsion or as a liposomal formulation.

Accordingly in one embodiment of the present invention there is provided a vaccine comprising a fusion protein or fusion partner protein as described herein and an adjuvant, for example as described above.

### Combination of PRAME and MAGE

In one embodiment of the present invention there is provided a composition comprising (a) an antigen component comprising a PRAME antigen or fusion protein as described herein and (b) an antigen component comprising a MAGE antigen or fusion protein as described herein. In one embodiment, the composition may further comprise an adjuvant as described herein.

The MAGE antigen for use in the combination may comprise the full length MAGE antigen. Alternatively, the MAGE antigen may comprise an immunogenic portion of MAGE in which 1, 2, 3, 4, 5, 6, 7, 8, 9, 10 or more amino acids may be deleted from or substituted in the amino acid sequence. In one embodiment of the present invention, 2 amino acids may be deleted from the N-terminus of the MAGE sequence. In one embodiment of the present invention in which the antigen is MAGE-A3 or an immunogenic portion thereof, the sequence of MAGE-A3 may be from amino acid 3 to 314 of MAGE-A3.

For the combination described above, either or both of the PRAME and/or MAGE antigens may be part of a fusion protein or proteins as described herein, or the antigens may be present in other fusion proteins or may be presented as antigen alone.

In one embodiment of the present invention there is provided a composition comprising a fusion protein comprising a PRAME antigen and fusion partner protein as described herein and a fusion protein comprising a MAGE-A3 antigen and fusion partner protein as described herein. In an alternative embodiment, the fusion protein comprising the MAGE-A3 antigen comprises or consists of the MAGE-A3 antigen and a fusion partner protein comprising approximately the first 109 amino acids of protein D, in which one or two or more amino acids from protein D are optionally substituted, and in which the signal sequence of protein D is optionally present, in addition to the first 109 amino acids of protein D.

The fusion proteins of the present invention may additionally optionally comprise one or more amino acids as "linkers" between the sequences of the antigen and the fusion partner protein or between the antigen and a His tail, if present. The amino acids may be unrelated to the sequences of the antigen and/or fusion partner.

Fusion proteins of the present invention, as described herein, may additionally comprise amino acids Met-Asp-Pro at the N-terminal end of the fusion protein sequence. The Met amino acid may be from the original protein D sequence or may be from an unrelated sequence.

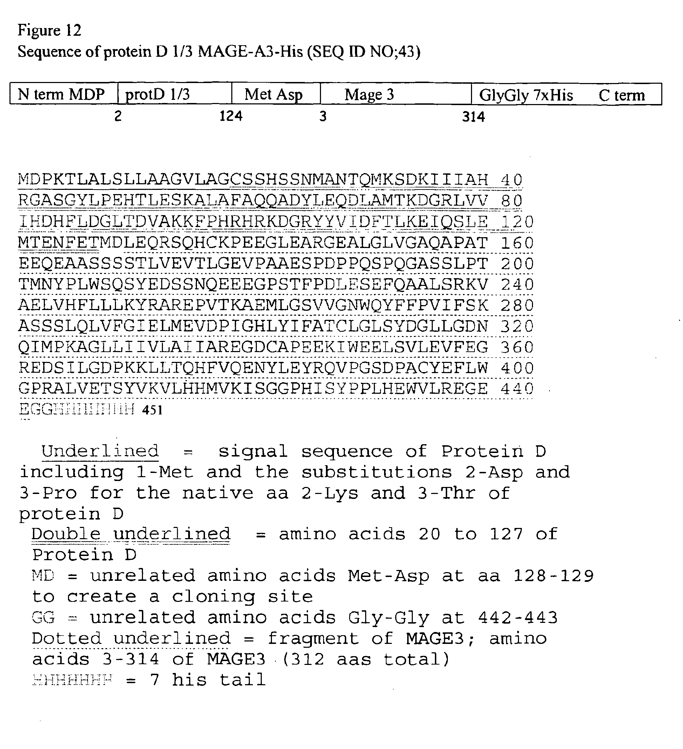

In one embodiment, the sequence of a fusion protein comprising MAGE-A3 and protein D for use in the present invention is shown in Figure 12 and SEQ ID NO:43.

The present invention also extends to methods of preparing said vaccines/ compositions.

### EXAMPLES

Four fusion constructs were prepared and will be referred to herein as Examples/constructs 1, 2, 3 and 4. A codon optimized construct was prepared from example 3 and is designated as example 3a herein. A codon optimized construct was prepared from example 4 and is designated as example 4a herein.

In Examples 3a and 4a the sequence in respect of the protein D portion of the molecule is the same. However, certain codons in the PRAME region were modified, to further improve expression and, in Example 3a, the linker between PRAME and the his tail has been removed.

**TABLE A Fusion Protein Structures and Plasmid Details of the Examples/Constructs 1 to 4**

| | **N-terminal-------------------------------------------------------------------------------------------------------- C Terminal** | | | | |
|---|---|---|---|---|---|
| Example/ Construct No. | amino acids inserted by cloning process | fusion partner | cancer antigen | Linker/His tail amino acid seq. | plasmid used |
| 1 | MDP | 20-127 protein D | PRAME | TSGHHHHHH | TCMP 14 |
| 2 | MDP | 20-127 protein D | PRAME | - | TCMP14 |
| 3 | MDP | 20-127 protein D | PRAME | LEHHHHHH | pET21 |
| 4 | MDP | 20-127 protein D | PRAME | - | pET21 |
| 3a | MDP | 20-127 protein D | PRAME | HHHHHH | pET26 |
| 4a | MDP | 20-127 protein D | PRAME | - | pET26 |

The fusion proteins of the above examples comprise the amino acids 20-127 of protein D. The amino acids Met, Asp and Pro were included at the N-terminal of the protein D fragment (ie amino acids MDP-20-127 Protein D). It is thought that these three additional amino acids may aid the stability of the protein and/or increase the level of the protein expression thereof. Amino acid 127 of protein D is fused to the N-terminal of full length PRAME (ie amino acid 127 of protein D is fused to N-terminal of PRAME). A histidine tag tail, to aid purification, was included in three of the six proteins. The exact sequence of the tail is dependent the plasmid used.

Three different types of plasmids, TCMP14 and pET21 or pET26 were constructed: for each plasmid, DNA encoding for fusion protein was included with and without a histidine tail.

Unless stated otherwise the general strategy below was used in the preparation of each of the examples.

### Cloning strategy for the generation of PD1/3-PRAME (with or without His-tag) recombinant protein using TCMP14 vector:

Amplification of the sequences presented in the plasmid TCMP14 were done using a three steps PCR strategy. The vector pHIC348 containing the DNA sequence encoding the entire protein D gene has been obtained from Dr. A. Forsgren, Department of Medical Microbiology, University of Lund, Malmö General Hospital, Malmö, Sweden. The DNA sequence of protein D has been published by Janson et al. (1991) {Janson H, LO Heden, A Grubb, M Ruan, & A Forsgren. 1991. Infect Immun 59:119-125}. The expression vector pMG81 is a derivative of pBR3 22, in which bacteriophage λ derived control elements for transcription and translation of foreign inserted genes were introduced (Shatzman et al., 1983) {Shatzman A, YS Ho, & M Rosenberg. 1983. Experimental Manipulation of Gene Expression. Inouya (ed) pp 1-14. Academic NY}. In addition, the Ampicillin resistance gene was exchanged with the Kanamycin resistance gene. The coding sequence for the portion of NS1 protein (amino acid 4 to 81) was substituted for a multiple cloning sites to get pMG81 MCS. The coding sequence for the 1/3 protein D (amino acid 20 to 127) was cloned into pMG81 MCS using BamHI and NcoI restriction sites to get pMG81-1/3PD. First, PCR amplification of the section corresponding to amino acid 20-127 of protein D was done using pMG81-1/3PD vector as template and oligonucleotide sense:
5' ATA TAA CAT ATG GAT CCA AGC AGC CAT TCA TCA AAT 3' (CAN008; SEQ ID NO: 18) and antisense:
5' CCA CAA ACG CCT TCG TTC CAT GGT TTC AAA GTT TTC TGT C 3' (CAN037; SEQ ID NO: 19).
PRAME cDNA obtained from the Ludwig Institute, Brussels, Belgium was inserted in the Bstx1-Not1 sites of the pCDNA1 vector (Invitrogen) to generate pCDNA-1-PRAME recombinant vector. PCR amplification of the section corresponding to amino acid of PRAME protein was done using pcDNA-1-PRAME vector (GSKBio) as template and oligonucleotide sense:
5' GAC AGA AAA CTT TGA AAC CAT GGA ACG AAG GCG TTT GTG G 3' (CAN036); SEQ ID NO: 20) and antisense:
5' AGA GAG ACT AGT CTA GTT AGG CAT GAA ACA GGG GCA CAG 3' (CAN029; SEQ ID NO: 21) or
5' GGA GGA ACT AGT GTT AGG CAT GAA ACA GGG GCA CAG 3' (CAN002; SEQ ID NO: 22) depending if a his-tail (CAN002) or not (CAN029) was added. The final PRAME sequence inserted in the TCMP14 plasmid was obtained following a PCR amplifications using the 1/3PD and PRAME gene templates that were generated in the preliminary steps for template and oligonucleotide sense: CAN008, and antisense: CAN029 or CAN002 depending if an his-tail was present (CAN002) or not (CAN029). NdeI at 5' end and SpeI at 3' end sites were also added for cloning of the fragment into TCMP 14 vector.

### Construction of the vector design to express the recombinant protein 1/3PD-PRAME with or without His-tag recombinant protein using pET21 vector:

A recombinant cDNA plasmid called pcDNA1-PRAME (as described in the previous strategy) containing the coding sequence for PRAME gene and the vector PMG81-1/3PD (as described in the previous strategy) containing the N-terminal portion of the protein D coding sequence were used. The cloning strategy included the following steps.
a) First, the 1/3PD sequence without secretion signal (secretion or signal sequence)was PCR amplified from plasmid PMG81-1/3PD using the oligonucleotide sense: 5' AGAGAGCATATGAGCAGCCATTCATCAAATATGGCG (CAN040; SEQ ID NO: 22), and the antisense:
   5' ACGTGGGCGGCCGCGGTTTCAAAGTTTTCTGTCATTTCTAA (CAN032; SEQ ID NO: 23);
   Ndel at the 5' end and Not1 at the 3' end sites were also added for cloning of the fragment into pET21b(+) vector.
b) The PRAME sequence was PCR amplified from plasmid pcDNA1-PRAME using the oligonucleotide sense:
   5' TTGTTGGCGGCCGCAATGGAACGAAGGCGTTTGTGGGGT (CAN033; SEQ NO: 25), and the antisense:
      5' GGAGGACTCGAGGTTAGGCATGAAACAGGGGCACAG (CAN034; SEQ ID NO: 26); Not1 at the 5' end and Xho1 at the 3' end sites were also added for cloning of the fragment into pET21b vector. This amplification resulted in the addition at the C-terminal of the protein of two amino acids, Leu and Glu, followed by 6 His in pET21b(+) plasmid. For the generation of the protein without His-tag, a stop codon (TAG) was added at the 3' end of the PRAME gene by using CAN033 and CAN035 (antisense: 5' GGAGGACTCGAGCTAGTTAGGCATGAAACAGGGGCACAG (CAN035; SEQ ID NO: XX) instead of CAN033 and CAN034.
c) Cloning into pET21b(+) plasmid (Invitrogen) of the above amplified fragments.
d) Removal of Not1 site between 1/3PD and PRAME by using QuikChange II Site-Directed Mutagenesis Kit (Stratagene) and the oligonucleotide sense:
   5' CAGAAAACTTTGAAACCATGGAACGAAGGCG (CAN106; SEQ NO: XX), and the antisense:
      5' cgccttcgttccatggtttcaaagttttctg (CAN107; SEQ ID NO: XX).
e) Addition of two amino acids Asp and Pro following the Met at position 1 at the N-terminal of the protein D 1/3 by mutagenesis and using the oligonucleotide sense:
   5' GGAGATATACATATGGATCCAAGCAGCCATTCATCAAATATGG (CAN104; SEQ ID NO: XX) and the antisense:
      5' CCATATTTGATGAATGGCTGCTTGGATCCATATGTATATCTCC (CAN105; SEQ ID NO: XX).

### Construction of the vector design to express the recombinant protein 1/3PD-PRAME codon optimized (without or with His tag) in pET26 vector:

The PRAME gene was codon optimized and cloned in pGA4 backbone with the addition of Not I and Xho1 sites in the 5' end and the 3' end of the optimized gene respectively.

This plasmid, named 0606420pGA4, was used to clone the gene in fusion with the PD1/3 in the pET26 vector using the following steps.
a) Removal of the Not1 / Xho1 fragment corresponding to the optimized PRAME sequence with a stop codon at the 3'end of the gene from 0606420pGA4 plasmid.
b) Cloning of the optimized PRAME fragment into a pET26b(+) plasmid which contain the 1/3PD previously cloned Nde1/Not1 with CAN040 and CAN032 oligonucleotides as described above and where Asp and Pro amino acids were added in N-terminal by mutagenesis method with CAN104 and CAN105 oligonucleotides.
c) Removal of the Not1 site by mutagenesis with oligonucleotides: sense
   5' GACAGAAAACTTTGAAACCATGGAACGTCGTCGTCTGTGG (CAN123; SEQ ID NO: XX) and antisense
   5' CCACAGACGACGACGTTCCATGGTTTCAAAGTTTTCTGTC (CAN124; SEQ ID NO: XX). This resulted in 1/3PD-PRAME codon optimized fusion protein without His-tail.
d) The plasmid was then used as a template for the generation of 1/3PD-PRAME codon optimized with 6 His plasmid. PCR amplification of the fusion protein was done with oligonucleotides sense
   5' GGAATTCCATATGGATCCAAGCAGCCATTC (CAN199; SEQ ID NO: XX) and a antisense
   5' GGAGCTCTCGAGTCAGTGGTGGTGGTGGTGGTGGTTCGGCATAAAGCACGGGC (CAN198; SEQ ID NO: XX); Nde1 at the 5' end, Xho1 at the 3' end sites followed by 6 His and a stop codon were also added for cloning of the fragment into pET26b(+) vector.
e) Cloning of the amplified fragment in pET26b(+) plasmid from Invitrogen.

For the production of the fusion protein, the DNA construct has been cloned into the expression vector TCMP14. This plasmid utilizes signals from lambda phage DNA to drive the transcription and translation of inserted foreign genes. The vector contains the lambda PL promoter PL, operator OL and two utilization sites (NutL and NutR) to relieve transcriptional polarity effects when N protein is provided (Gross et al., 1985. Mol. & Cell. Biol. 5:1015).

The plasmid expressing the pD-PRAME fusion protein was designed so the PRAME amino acids were added to the C-terminal of a 108 amino acids derivative of pD without its signal sequence (secretion or signal sequence) (i.e. residues 20-127). To this construction, three unrelated amino acids (Met and Asp and a Proline) were added at the N-terminal of the derivative ofpD, and for certain constructions a his tail at the C-terminal of the PRAME amino acids was included (see table A above). This construct could alternatively be described as containing 109 amino acids derivative of pD, if the N-terminal Met is considered to come from the pD sequence.

### Host Strain and Transformation

Hosts from *E. Coli* strain AR58 (Mott et al, Proc. Natl. Acad. Sci. USA, vol 82, pp 88-92, January 1985, Biochemistry) were transformed with plasmid DNA for Examples/ constructs 1 and 2.

The AR58 lysogenic *E. coli* strain used for the production of Examples/constructs 1 and 2 is a derivative of the standard NIH *E.coli* K12 strain N99 (F- su- galK2, lacZ- thr-). It contains a defective lysogenic lambda phage (galE::TN10, 1 Kil- cI857 DH1). The Kilphenotype prevents the shut off of host macromolecular synthesis. The cI857 mutation confers a temperature sensitive lesion to the cI repressor. The DH1 deletion removes the lambda phage right operon and the hosts bio, uvr3, and chlA loci. The AR58 strain was generated by transduction of N99 with a P lambda phage stock previously grown on an SA500 derivative (galE::TN10, 1 Kil- cI857 DH1). The introduction of the defective lysogen into N99 was selected with tetracycline by virtue of the presence of a TN10 transposon coding for tetracyclin resistance in the adjacent galE gene. N99 and SA500 are E.coli K12 strains derived from Dr. Martin Rosenberg's laboratory at the National Institutes of Health.

Vectors containing the PL promoter, are introduced into an E. coli lysogenic host to stabilize the plasmid DNA. Lysogenic host strains contain replication-defective lambda phage DNA integrated into the genome (Shatzman et al., 1983; In Experimental Manipulation of Gene Expression. Inouya (ed) pp 1-14. Academic Press NY). The lambda phage DNA directs the synthesis of the cI repressor protein which binds to the OL repressor of the vector and prevents binding of RNA polymerase to the PL promoter and thereby transcription of the inserted gene. The cI gene of the expression strain AR58 contains a temperature sensitive mutation so that PL directed transcription can be regulated by temperature shift, i.e. an increase in culture temperature inactivates the repressor and synthesis of the foreign protein is initiated. This expression system allows controlled synthesis of foreign proteins especially of those that may be toxic to the cell (Shimataka & Rosenberg, 1981. Nature 292:128).

Hosts from *E. Coli* strain BLR (DE3) Novagen, WI, USA (catalogue number: 69053-4) BLR (DE3) Novagen, WI, USA (catalogue number: 69053-4) BLR is a *recA⁻* derivative of BL21 that improves plasmid monomer yields and may help stabilize target plasmids containing repetitive sequences or whose products may cause the loss of the DE3 prophage (1, 2) were transformed with plasmid DNA from examples/constructs 3 and 4.

Each of transformation was carried out by standard methods with CaCl₂-treated cells (Hanahan D. « Plasmid transformation by Simanis. » In Glover, D. M. (Ed), DNA cloning. IRL Press London. (1985): p. 109-135.).

### Growth and induction of bacterial host strain

### • Culture

Bacteria were grown-on 20 ml of Luria-Bertani (LB) broth (BD) + 1% (w/v) glucose (Laboratoire MAT, catalogue number: GR-0101) + antibiotic(Carbenicillin 100 µg/ml for pET21b, kanamycin 40 µg/ml for TCMP14). Cultures were incubated at 33°C, for AR58 cells and at 37°C, for BLR (DE3) cells until an O.D.₆₀₀ₙₘ around 0.8.

### • Induction

At O.D.₆₀₀nm around 0.8, the cultures BLR (DE3) were induced at 1 mM isopropyl β-D-1-thiogalactopyranoside (IPTG; EMD Chemicals Inc., catalogue number: 5815) and incubated for 2 hours or 3 hours at 37°C although solubility may be increased if a lower temperature is used.

At O.D.₆₀₀ₙₘ around 0.8, the cultures AR58 were induced by heat activation at 37°C and incubated for 7 hours.

The bacterial growth was adequate for the two expression systems.

### • Extraction and Purification of the Protein

Upon expression of the polypeptide in culture, cells are typically harvested by centrifugation then disrupted by physical or chemical means (if the expressed polypeptide is not secreted into the media) and the resulting crude extract retained to isolate the polypeptide of interest. BugBuster^{™} Protein Extraction Reagent is used under conditions recommended by the suppliers (Novagen).

### PD1/3-Prame-his protein purification

E. coli cell paste was resuspended in 20 mM Tris buffer pH 8.5 then passed through homogenizer system (Panda from Niro Soavi S.p.A. - 2 passes - 750 bars). After addition of 2 mM MgCl₂ and Benzonase (50 U/ml), homogenate was incubated 1 hour at room temperature (RT) under gentle agitation then centrifuged 30 minutes at 15900 g and RT. Resulting pellet was resuspended in 20 mM Tris buffer pH 8.5 containing 1% Sodium Dodecyl Sulphate (SDS) and 60 mM Glutathione and incubated 30 minutes at RT under gentle agitation. After centrifugation 30 minutes at 15900 g and RT, pellet was discarded.

Centrifugation supernatant was 10-fold diluted in 20 mM Tris buffer containing 6.66 M Urea, 0.333 M sodium chloride (NaCl) and 11.11 mM Imidazole and then subjected to chromatographic separation on a Nickel ion metal affinity column (IMAC Sepharose 6 FF - GE Healthcare) equilibrated in a 20 mM Tris buffer pH 8.5 containing 0.1% SDS, 6.0 M Urea, 0.3 M NaCl and 10 mM Imidazole. After washing of the column with 20 mM Tris buffer pH 8.5 containing 0.5% Sarcosyl, 6.0 M Urea, 0.3 M NaCl and 10 mM Imidazole, antigen was eluted from the column by increasing the concentration of Imidazole up to 40 mM in the same washing buffer. After addition of phosphate up to 50 mM, antigen positive eluate was passed through a Macro-Prep Ceramic Hydroxyapatite type II column (Bio-Rad) equilibrated in a 20 mM Tris buffer pH 8.5 containing 50 mM phosphate, 0.5% Sarcosyl, 6.0 M Urea and 0.3 M NaCl. Hydroxyapatite flow-through containing the antigen was then diafiltered against 5 mM Borate buffer pH 9.8 containing 3.15% Sucrose on an Omega 30 kDa membrane (Pall). Ultrafiltration retentate was sterilized by filtration through a 0.45/0.22 µm Cellulose acetate membrane (Sartorius). Purified material was stored at -70°C.

An alternative purification process has also been used, which differs from the above process in the following steps:
- No benzonase treatment
- No shift from SDS to sarcosyl on IMAC column (SDS from extraction up to HA step)
- The buffer used for the diafiltration was 5 mM Tris buffer pH 8.5 - 0.5 M Arginine.
This alternative purification process resulted in incomplete SDS removal with residual value around 0.05 and 0.085%.

### • Purification

The expressed recombinant proteins were purified from supernatant fractions obtained after centrifugation of induced *E. coli* using a His-Bind metal chelation resin (QIAgen, Chatsworth, CA) according to the instructions from the resin manufacturer.

### Characterisation of the Protein

### SDS-Page:

Gel: NuPAGE 4-12 % Bis-Tris Gel 1.0mm 15 or 26 wells (Invitrogen catalog number: NP0323BOX)

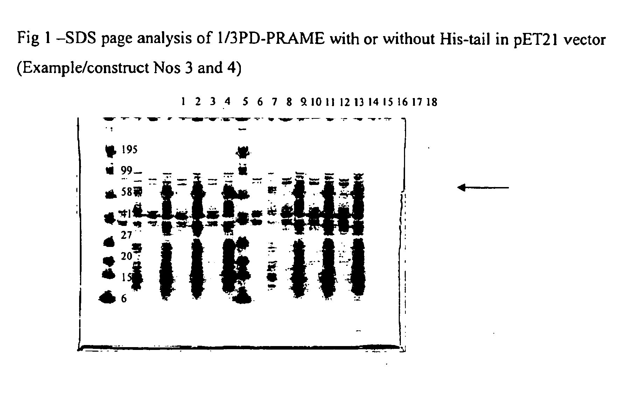

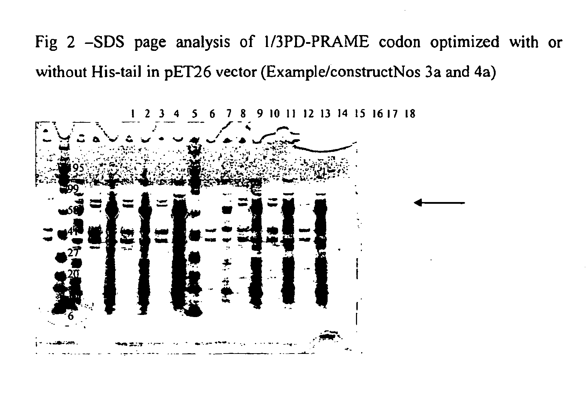

See Figure 1 and 2 below, which show SDS page analysis of Example 3 and 4 and 3a and 4a respectively, wherein the different recombinant 1/3pD-PRAME proteins with or without his-tag migrate on gel at an apparent molecular weight of ~70kDa. The recombinant proteins are found as an inclusion bodies in the E coli cell lysate, after induction.

Preparation of samples, buffers and migration conditions were done under conditions recommended by the suppliers (Invitrogen). 10µl of all preparations were loaded (before induction (BI) and after induction (AI)) in wells corresponding to 100 µl of culture equivalent.

Fig 1 legend: SDS page analysis after Coomassie-blue staining of recombinant 1/3PD-PRAME after IPTG induction of *E. coli* BLR DE3 strain transformed with recombinant pET21. An equivalent of 100µL of culture after 2 hours of induction in BLR DE3 strain with 1mM IPTG at 25, 30 or 37°C has been loaded on gel. Clone #3 (1/3PD-PRAME / pET21) and Clone #4 (1/3PD-PRAME-His / pET21) are presented on gel before (BI) and after (AI) inductions in soluble (supernatant) and non-soluble (pellet) fractions. Lane 1 and 10: Standard Broad Range prestain (BioRad Cat#161-0318), lane 2 (clone #3, BI, supernatant), lane 3 (clone #3, BI, pellet), lane 4 (clone #3, AI, 25°C, supernatant), lane 5 (clone #3, AI, 25°C, pellet), lane 6 (clone #3, AI, 30°C, supernatant), lane 7 (clone #3, AI, 30°C, pellet), lane 8 (clone #3, AI, 37°C, supernatant), lane 9 (clone #3, AI, 37°C, pellet), lane 11 (clone #4, BI, supernatant), lane 12 (clone #4, BI, pellet), lane 13 (clone #4, AI, 25°C, supernatant), lane 14 (clone #4, AI, 25°C, pellet), lane 15 (clone #4, AI, 30°C, supernatant), lane 16 (clone #4, AI, 30°C, pellet), lane 17 (clone #4, AI, 37°C, supernatant), lane 18 (clone #4, AI, 37°C, pellet).

Fig 2 legend: SDS page analysis after Coomassie-blue staining of recombinant 1/3PD-PRAME after IPTG induction of *E. coli* BLR DE3 strain transformed with recombinant pET26. An equivalent of 100µL of culture after 2 hours of induction in BLR DE3 strain with 1mM IPTG at 25, 30 or 37°C has been loaded on gel. Clone #3a (1/3PD-PRAME codon optimized / pET26) and Clone #4a (1/3PD-PRAME-His codon optimized / pET26) are presented on gel before (BI) and after (AI) inductions in soluble (supernatant) and non-soluble (pellet) fractions. Lane 2 and 10: Standard Broad Range prestain (BioRad Cat#161-0318), lane 1 (clone #3a, BI, supernatant), lane 3 (clone #3a, BI, pellet), lane 4 (clone #3a, AI, 25°C, supernatant), lane 5 (clone #3a, AI, 25°C, pellet), lane 6 (clone #3a, AI, 30°C, supernatant), lane 7 (clone #3a, AI, 30°C, pellet), lane 8 (clone #3a, AI, 37°C, supernatant), lane 9 (clone #3a, AI, 37°C, pellet), lane 11 (clone #4a, BI, supernatant), lane 12 (clone #4a, BI, pellet), lane 13 (clone #4a, AI, 25°C, supernatant), lane 14 (clone #4a, AI, 25°C, pellet), lane 15 (clone #4a, AI, 30°C, supernatant), lane 16 (clone #4a, AI, 30°C, pellet), lane 17 (clone #4a, AI, 37°C, supernatant), lane 18 (clone #4a, AI, 37°C, pellet).

### Western Blot

Membranes were blocked for 30 minutes at 37°C, 60RPM using 3% milk/PBS 1X fresh solution. After the blocking incubation, primary antibodies were added (rabbit anti-PRAME; GSK Biologicals SA) at dilution 1:5000 or α-6X His tag (AbCam) at dilution 1:3000 in 3% milk/PBS 1X fresh solution for 1 hour at 37°C, 60RPM. After that, membranes were washed three times 5 minutes at room temperature using 0.02% Tween20/PBS 1X. Secondary antibodies were added (perox donkey anti-IgG (H+L) rabbit (Jackson laboratory) at dilution 1:20 000 using 3% milk/PBS 1X fresh solution. Membranes were incubated for I hour at 37°C, 60RPM. After that, membranes were washed three times 5 minutes at room temperature using 0.02% Tween20/PBS 1X before the membrane expositions to peroxydase substrate (KH₂PO₄, 10mM; (NH₄)₂SO₄, 10mM; O-dianisidine, 0.01% & hydrogen peroxide 0.045%) or alkaline phosphatase substrate (Sigma Fast) following the supplier's recommendations.

### Molecular analysis:

### Example/construct 1

| **Analysis** | **Entire Protein** |
|---|---|
| Length | 629 aa |
| Molecular Weight | 71629.96 m.w. |
| 1 microgram = | 13.961 pMoles |
| Molar Extinction coefficient | 67680 |
| 1 A[280] corr. to | 1.06 mg/ml |
| A[280] of 1 mg/ml | 0.94 AU |
| Isoelectric Point | 6.41 |
| Charge at pH 7 | -5.84 |
| Isoelectric Point | 6.41 |
| Charge at pH 7 | -5.84 |

### Example/construct 2

| **Analysis** | **Entire Protein** |
|---|---|
| Length | 620 aa |
| Molecular Weight | 70561.90 m.w. |
| 1 microgram = | 14.172 pMoles |
| Molar Extinction coefficient | 67680 |
| 1 A[280] corr. to | 1.04 mg/ml |
| A[280] of 1 mg/ml | 0.96 AU |
| Isoelectric Point | 6.28 |
| Charge at pH 7 | -6.36 |

### Example/construct 3

| **Analysis** | **Entire Protein** |
|---|---|
| Length | 628 aa |
| Molecular Weight | 71627.01 m.w. |
| 1 microgram = | 13.961 pMoles |
| Molar Extinction coefficient | 67680 |
| 1 A[280] corr. to | 1.06 mg/ml |
| A[280] of 1 mg/ml | 0.94 AU |
| Isoelectric Point | 6.34 |
| Charge at pH 7 | -6.84 |

### Example/construct 4

| **Analysis** | **Entire Protein** |
|---|---|
| Length | 620 aa |
| Molecular Weight | 70561.90 m.w. |
| 1 microgram = | 14.172 pMoles |
| Molar Extinction coefficient | 67680 |
| 1 A[280] corr. to | 1.04 mg/ml |
| A[280] of 1 mg/ml | 0.96 AU |
| Isoelectric Point | 6.28 |
| Charge at pH 7 | -6.36 |

### Example 5

### Evaluation of the production of proteins with or without the secretion signal (secretion or signal sequence) of the protein D 1/3 in the fusion protein.

**Table B**

| **Protein** | **Expression level** |
|---|---|
| PD1/3-PRAME **with** secretion signal | + |
| PD1/3-PRAME **without** secretion signal | +++ |

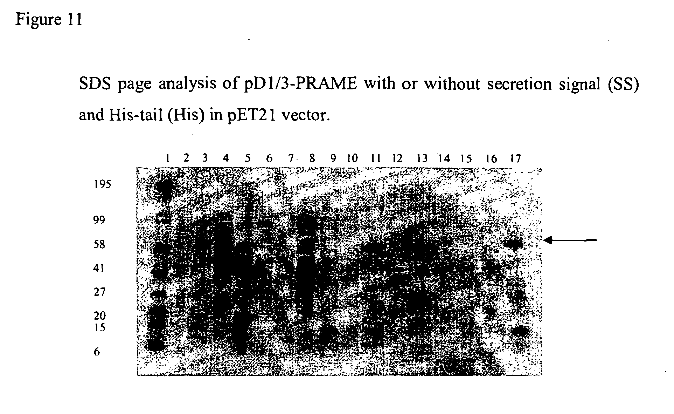

**Fig 11**: SDS page analysis after Coomassie-blue staining of recombinant 1/3PD-PRAME with or without secretion signal after IPTG induction of *E. coli* BL21 DE3 strain transformed with recombinant pET21. An equivalent of 100µL of culture after 3 hours of induction in BL21 DE3 strain with 1mM IPTG at 37°C has been loaded on gel. Those constructs are presented on gel before (BI) and after (AI) inductions in soluble (supernatant) and non-soluble (pellet) fractions. Lane 1: Standard Broad Range prestain (BioRad Cat#161-0318), lane 2 (pD1/3-PRAME + SS, BI, supernatant), lane 3 (pD1/3-PRAME + SS, BI, pellet), lane 4 (pD1/3-PRAME + SS, AI, supernatant), lane 5 (pD1/3-PRAME + SS, Al, pellet), lane 6 (pD1/3-PRAME + SS + His, BI, supernatant), lane 7 (pD1/3-PRAME + SS + His, BI, pellet), lane 8 (pD1/3-PRAME + SS + His, AI, supernatant), lane 9 (pD1/3-PRAME + SS + His, AI, pellet), lane 10 (pD1/3-PRAME w/o SS, BI, supernatant), lane 11 (pD1/3-PRAME w/o SS, BI, pellet), lane 12 (pD1/3-PRAME w/o SS, AI, supernatant), lane 13 (pD1/3-PRAME w/o SS, AI, pellet), lane 14 (pD1/3-PRAME w/o SS + His, BI, supernatant), lane 15 (pD1/3-PRAME w/o SS + His, BI, pellet), lane 16 (pD1/3-PRAME w/o SS + His, AI, supernatant), lane 17 (pD1/3-PRAME w/o SS + His, AI, pellet).

### Example 6: Immunogenicity of PD-PRAME-His formulated in AS01B or AS15: dose range of antigen in a constant dose of adjuvant.

Aim: dose-range of antigen to select the best dose to use in preclinical experiments

### Protocol:

6 groups of 12 CB6F1 mice received intra-muscular (IM) injections at day 0 and 14 of:
1. PBS
2. PRAME (50* µg) in AS01B or AS 15
3. PRAME (10µg) in AS01B or AS15
4. PRAME (2µg) in AS01B or AS 15
5. PRAME (0.4µg) in AS01B or AS 15
6. PRAME (0.08µg) in AS01B or AS15
* 44.7µg actually administered instead of the 50µg intended dose

AS01B is a liposomal adjuvant formulation comprising QS21 and 3D-MPL; AS15 is a liposomal adjuvant formulation comprising QS21, 3D-MPL and CpG.

The construct used in this example was Example/Construct 3a (pET26 with a His tail), provided in 5 mM Tris buffer pH 8.5 - 0.5 M Arginine. Protein provided in a borate buffer with sucrose may also be used.

### Read-outs:

• Intracellular Cytokine Staining (ICS) 14 days post 2 injections after in vitro restimulation of spleen cells (4 pools of 3 mice per group) with the pool of peptides PRAME at I µg/ml/peptide (15-mer)

### CD4 response (AS01B adjuvant)

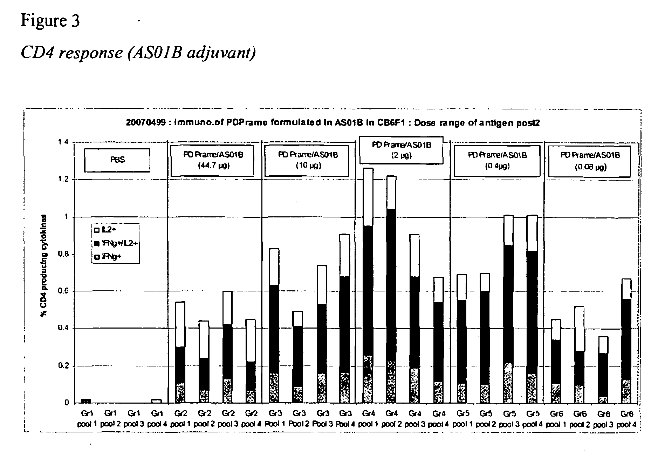

Results of ICS for CD4 cytokines for the AS01B adjuvant are shown in Figure 3. In this experiment it may be concluded that the best dose of PRAME antigen to induce a CD4 response in AS01B under these conditions seems to be 2µg.

### CD8 response (AS01B adjuvant)

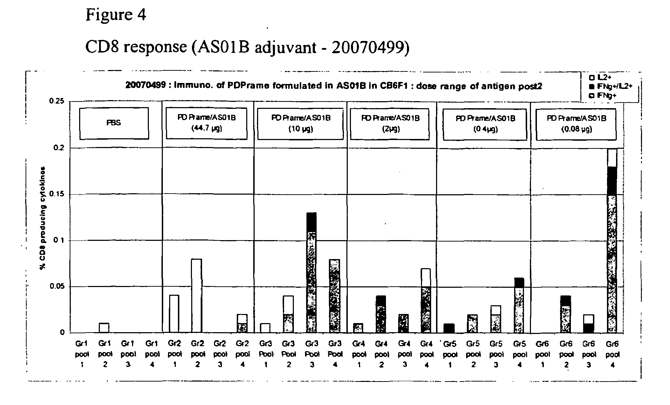

Results of ICS for CD8 cytokines for the AS01B adjuvant are shown in Figure 4. The data appear to show a very low CD8 response and heterogeneity of the response intra-group.

### CD4 response (AS15 adjuvant)

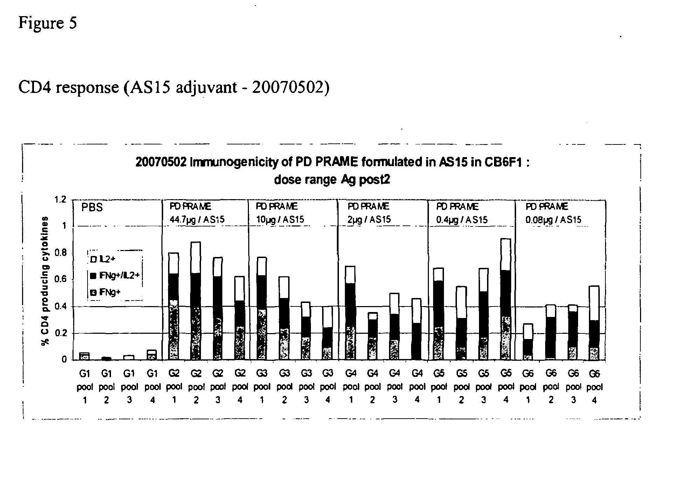

Results of ICS for CD4 cytokines for the AS15 adjuvant are shown in Figure 5. These data appear to show that a similar CD4 response was induced with 44µg, 10µg, 2µg and 0.4µg of PRAME formulated in AS15; with a decreased response induced with 0.08µg PRAME

### CD8 response (AS15 adjuvant)

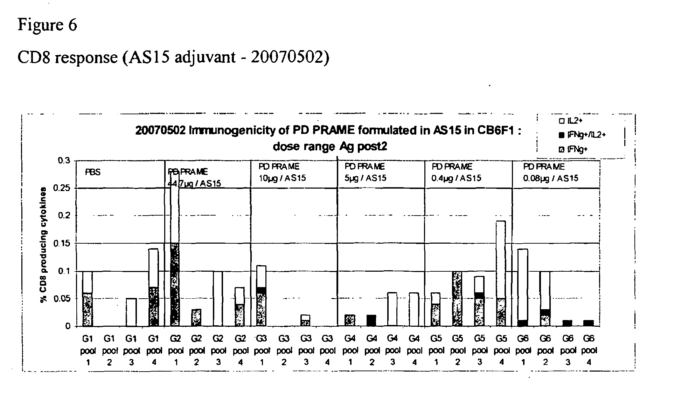

Results of ICS for CD8 cytokines for the AS15 adjuvant are shown in Figure 6. These data appear to show no CD8 response (background in the PBS group)

### Example 7

In summary, for the inventions described herein, the following summary may be used to described specific constructs of PD1/3-PRAME that have so far been generated:

### Constructs used for PD1/3-PRAME

- No signal sequence of Protein D are included (amino acids 2 to 19 of protein D)
- The Methionine of Protein D is included (AA 1 of the protein D)
- Two unrelated AA (Asp and Pro) are substituted for amino acids 2-Lys and 3-Leu of Protein D
- The first 109 AA of protein D after the signal sequence of protein D are included (109 amino acids including the first Met in N-term ± AA20 to 127 of the protein D)
- AA 1 - 509 of PRAME are included (full length original sequence of PRAME)
- With or without a His tail composed of one of the following:
   - Three unrelated amino acid (Thr, Ser and Gly) + 6 His residues for cloning in TCMP 14 plasmid; or
   - Two unrelated amino acid (Leu and Glu) + 6 His residues for cloning in pET21 plasmid; or
   - 6 His residues for cloning in pET26 plasmid.

### pD1/3 - PRAME +/- His tail protein :

| | pD1/3 20 - 127 | PRAME 1 - 509 | |
|---|---|---|---|
| N term MDP | pD 1/3 | PRAME | C term |
| N term MDP | pD 1/3 | PRAME | TSG 6xHis (in TCMP14) C term |
| N term MDP | pD 1/3 | PRAME | LE 6xHis (in pET21) C term |
| N term MDP | pD 1/3 | PRAME | 6xHis (in pET26) C term |

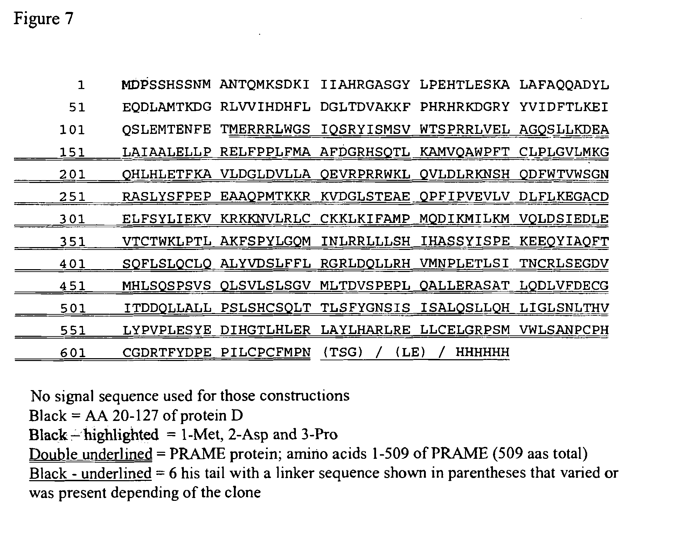

A marked up amino acid sequence of examples of constructs of the present invention is shown in Figure 7

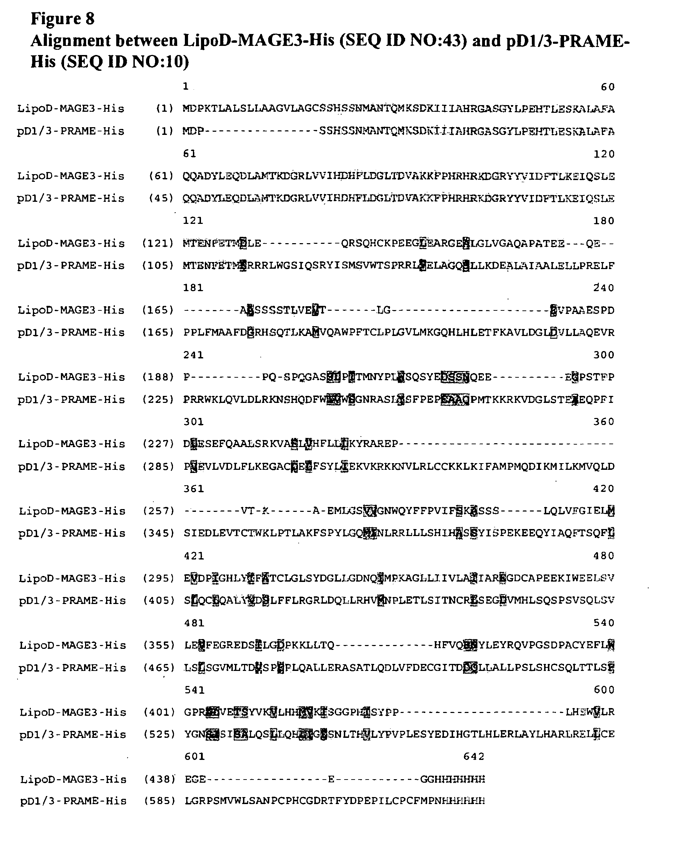

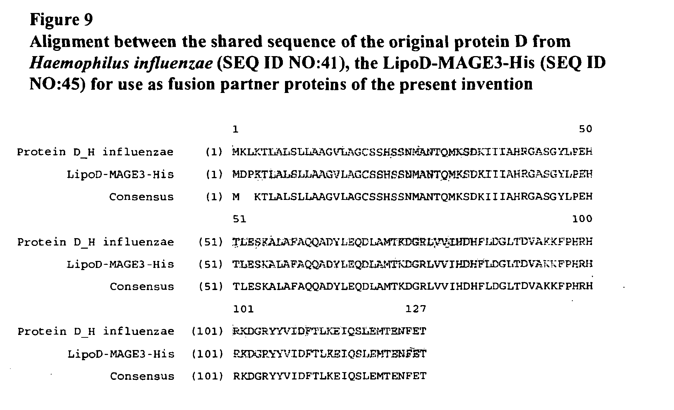

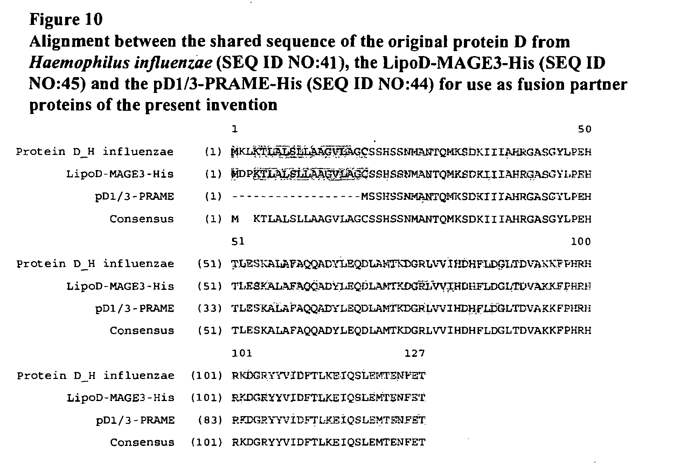

Alignments of the following constructs are shown in Figures 8, 9 and 10:
Alignment between LipoD-MAGE3-His and D1/3-PRAME-His (Figure 8)
Alignment between the shared sequence of the original protein D from *Haemophilus influenzae* and the LipoD-MAGE3-His (Figure 9)
Alignment between the shared sequence of the original protein D from *Haemophilus influenzae,* the LipoD-MAGE3-His and the pD1/3-PRAME-His (Figure 10)

### Formulation of Vaccine preparation using fusion proteins:

The fusion proteins of the invention can be formulated into vaccines which are either adjuvanted or not. In one embodiment, as an adjuvant, the formulation may comprise a mixture of 3 de -O-acylated monophosphoryl lipid A (3D-MPL) and QS21 in an oil/water emulsion. The adjuvant system SBAS2 has been previously described WO 95/17210. The adjuvant for use in the present invention may alternatively comprise 3 de -O-acylated monophosphoryl lipid A (3D-MPL), QS21 and CpG in an oil-in water formulation or in a liposomal formulation.

**3D-MPL:** is an immunostimulant derived from the lipopolysaccharide (LPS) of the Gram-negative bacterium *Salmonella minnesota.* MPL has been deacylated and is lacking a phosphate group on the lipid A moiety. This chemical treatment dramatically reduces toxicity while preserving the immunostimulant properties (Ribi, 1986).
It is believed that 3D-MPL combined with various vehicles may strongly enhance both the humoral and a TH1 type of cellular immunity.

**QS21**: is a natural saponin molecule extracted from the bark of the South American tree Quillaja saponaria Molina. A purification technique developed to separate the individual saponines from the crude extracts of the bark, permitted the isolation of the particular saponin, QS21, which is a triterpene glycoside demonstrating stronger adjuvant activity and lower toxicity as compared with the parent component. QS21 has been shown to activate MHC class I restricted CTLs to several subunit Ags, as well as to stimulate Ag specific lymphocytic proliferation (Kensil, 1992).

It is thought that there may be a synergistic effect of combinations of MPL and QS21 in the induction of both humoral and TH1 type cellular immune responses.

**The oil/water emulsion** comprises an organic phase made of 2 oils (a tocopherol and squalene), and an aqueous phase of PBS containing Tween 80 as emulsifier. The emulsion comprised 5% squalene 5% tocopherol 0.4% Tween 80 and had an average particle size of 180 nm and is known as SB62 (see WO 95/17210). The resulting oil droplets should have a size of approximately 180 nm.

The adjuvant for use in the present invention may be formulated as a combination of MPL and QS21, in an oil/water emulsion or in a liposomal formulation. This preparation should be delivered in vials of 0.7 ml to be admixed with lyophilised antigen or fusion protein (vials containing from 30 to 300 µg antigen).
Immunostimulatory oligonucleotides may also be used. Examples oligonucleotides for use in adjuvants or vaccines of the present invention include CpG containing oligonucleotides, generally containing two or more dinucleotide CpG motifs separated by at least three, more often at least six or more nucleotides. A CpG motif is a cytosine nucleotide followed by a guanine nucleotide. The CpG oligonucleotides are typically deoxynucleotides. In one embodiment the internucleotide in the oligonucleotide is phosphorodithioate, or more preferably a phosphorothioate bond, although phosphodiester and other internucleotide bonds are within the scope of the invention. Also included within the scope of the invention are oligonucleotides with mixed internucleotide linkages. Methods for producing phosphorothioate oligonucleotides or phosphorodithioate are described in US5,666,153, US 5,278,302 and WO 95/26204.

Examples of oligonucleotides are as follows:
TCC ATG ACG TTC CTG ACG TT (CpG 1826; SEQ ID NO: 36)
TCT CCC AGC GTG CGC CAT (CpG 1758; SEQ ID NO: 37)
ACC GAT GAC GTC GCC GGT GAC GGC ACC ACG TCG TCG TTT TGT CGT TTT GTC GTT (CpG 2006; SEQ ID NO: 38)
TCC ATG ACG TTC CTG ATG CT (CpG 1668; SEQ ID NO: 39)
TCG ACG TTT TCG GCG CGC GCC G (CpG 5456; SEQ ID NO: 40),
the sequences may contain phosphorothioate modified internucleotide linkages.

Alternative CpG oligonucleotides may comprise one or more sequences above in that they have inconsequential deletions or additions thereto.

The CpG oligonucleotides may be synthesized by any method known in the art (for example see EP 468520). Conveniently, such oligonucleotides may be synthesized utilising an automated synthesizer.

In one embodiment of the present invention an adjuvant combination for use in the invention includes one or more of the following components: 3D-MPL and QS21 (EP 0 671 948 B1); oil in water emulsions comprising 3D-MPL and QS21 (WO 95/17210, WO 98/56414); or 3D-MPL formulated with other carriers (EP 0 689 454 B1). Other adjuvant systems that may be used in the present invention comprise a combination of 3D-MPL, QS21 and a CpG oligonucleotide as described in US6558670 and US6544518.

The final vaccine may be obtained after reconstitution of the lyophilized formulation.

### References:

1. A. Roca (U. of Wisconsin), personal communication.
2. Studier, F.W. (1991) J. Mol. Biol. 219, 37-44.
3. Jan H. Kesslera et al The Journal of Experimental Medicine, Volume 193, Number 1, January 1, 2001 73-88,
4. Ikeda H et al Immunity, Feb; 6(2): 1997, 199-208

SEQ ID NO: 1
   DNA sequence for Example 1
SEQ ID NO: 2
   Amino Acid Sequence for Example 1
SEQ ID NO: 3
   DNA Sequence for example 2
SEQ ID NO: 4
   AMINO ACIDS SEQUENCE EXAMPLE 2
SEQ ID NO:5
   DNA sequence for Example 3
SEQ ID NO: 6
   Amino acids sequence: for Example 3
SEQ ID NO: 7.
   DNA sequence for Example 4:
SEQ ID NO: 8
   Amino Acid Sequence for Example 4
SEQ ID NO: 9
   Codon optimised DNA sequence for example 3a
SEQ ID NO: 10
   Amino Acid Sequence for Example 3a
SEQ ID NO: 11
   Codon optimised DNA sequence for Example 4a
SEQ ID NO: 12
   Amino Acid Sequence for Example 4a

## Claims

1. A fusion protein comprising:
(a) cancer testis antigen PRAME;
(b) a heterologous fusion partner protein derived from protein D, wherein the fusion partner protein comprises amino acids 20 to 127 of protein D; and
(c) additional amino acids Met-Asp-Pro at the N-terminal end of the fusion protein sequence
wherein the heterologous fusion partner protein derived from protein D, (b), is fused to the N-terminus of PRAME

2. A fusion protein according to claim 1 further comprising an affinity tag.

3. A fusion protein according to claim 2, wherein the affinity tag is a histidine tail.

4. A fusion protein according to any of preceding claims additionally comprising one or more linker sequences between the fusion partner protein and PRAME; or between the fusion partner protein and affinity tag; or between PRAME and affinity tag.

5. A nucleic acid sequence encoding a fusion protein as claimed in any of claims 1 to 4.

6. A vector comprising the nucleic acid sequence of claim 5.

7. An isolated host cell transformed with the vector of claim 6.

8. A vaccine containing a fusion protein as claimed in any of claims 1 to 4 or a nucleic acid as claimed in claim 5 or a vector as claimed in claim 6.

9. A vaccine as claimed in claim 8 additionally comprising an adjuvant, and/or immunostimulatory cytokine or chemokine.

10. A vaccine as claimed in claim 9 wherein the adjuvant comprises 3D-MPL, QS21 and/or a CpG oligonucleotide.

11. A vaccine as claimed in any of claims 8 to 10 for use in medicine.

12. Use of a protein or nucleic acid or vector according to any one of claims 1 to 6 for the manufacture of a vaccine for immunotherapeutically treating a patient suffering cancer.

13. A process for producing a fusion protein comprising the step of expressing in an isolated cell a fusion protein according to any of claims 1 to 4.

14. A process according to claim 13 in which the cell is a bacterium.

15. A process according to claim 14 in which the bacterium is E. coli.

16. A process according to claim 16 further comprising the step of lysing the cell and purifying the expressed fusion protein from the lysed cells.

17. A fusion protein obtained by or obtainable by the process of any of claims 13 to 16.

18. The use according to claim 12 in which the cancer is selected from melanoma, breast, bladder, lung cancer such as NSCLC, sarcoma, ovarian cancer, head and neck cancer, renal cancer, colorectal carcinoma, multiple myeloma, leukemia including acute leukemia and oesophageal carcinoma.

## Patentansprüche

1. Fusionsprotein, umfassend:
(a) das Krebs-Hoden-Antigen PRAME;
(b) ein heterologes Fusionspartnerprotein, das von Protein D abgeleitet ist, wobei das Fusionspartnerprotein die Aminosäuren 20 bis 127 von Protein D umfasst; und
(c) zusätzliche Aminosäuren Met-Asp-Pro am N-terminalen Ende der Fusionsproteinsequenz,
wobei das heterologe Fusionspartnerprotein, das von Protein D abgeleitet ist, (b), an den N-Terminus von PRAME gekoppelt ist.

2. Fusionsprotein gemäß Anspruch 1, ferner umfassend einen Affinitäts-Tag.

3. Fusionsprotein gemäß Anspruch 2, wobei der Affinitäts-Tag ein Histidin-Schwanz ist.

4. Fusionsprotein gemäß mindestens einem der vorhergehenden Ansprüche, zusätzlich umfassend eine oder mehrere Verbindungssequenzen zwischen dem Fusionspartnerprotein und PRAME; oder zwischen dem Fusionspartnerprotein und dem Affinitäts-Tag; oder zwischen PRAME und dem Affinitäts-Tag.

5. Nukleinsäuresequenz, die ein Fusionsprotein gemäß mindestens einem der Ansprüche 1 bis 4 kodiert.

6. Vektor, der die Nukleinsäuresequenz gemäß Anspruch 5 umfasst.

7. Isolierte Wirtszelle, die mit dem Vektor gemäß Anspruch 6 transformiert ist.

8. Impfstoff, enthaltend ein Fusionsprotein gemäß mindestens einem der Ansprüche 1 bis 4 oder eine Nukleinsäure gemäß Anspruch 5 oder einen Vektor gemäß Anspruch 6.

9. Impfstoff gemäß Anspruch 8, zusätzlich umfassend ein Adjuvans und/oder ein immunstimmulatorisches Zytokin oder Chemokin.

10. Impfstoff gemäß Anspruch 9, wobei das Adjuvans 3D-MPL, QS21 und/oder ein CpG-Oligonukleotid umfasst.

11. Impfstoff gemäß mindestens einem der Ansprüche 8 bis 10 zur Verwendung in der Medizin.

12. Verwendung eines Proteins oder einer Nukleinsäure oder eines Vektors gemäß mindestens einem der Ansprüche 1 bis 6 zur Herstellung eines Impfstoffs zur immuntherapeutischen Behandlung eines Patienten, der an Krebs leidet.

13. Verfahren zur Herstellung eines Fusionsproteins, umfassend den Schritt des Exprimierens eines Fusionsproteins gemäß mindestens einem der Ansprüche 1 bis 4 in einer isolierten Zelle.

14. Verfahren gemäß Anspruch 13, wobei die Zelle ein Bakterium ist.

15. Verfahren gemäß Anspruch 14, wobei das Bakterium E. coli ist.

16. Verfahren gemäß Anspruch 16, ferner umfassend den Schritt des Lysierens der Zelle und das Reinigen des exprimierten Fusionsproteins aus den lysierten Zellen.

17. Fusionsprotein, erhalten durch oder erhältlich durch das Verfahren gemäß mindestens einem der Ansprüche 13 bis 16.

18. Verwendung gemäß Anspruch 12, wobei der Krebs ausgewählt ist aus Melanom, Brust-, Blasen-, Lungenkrebs wie beispielsweise nicht-kleinzelliges Bronchialkarzinom (NSCLC), Sarkom, Ovarialkrebs, Kopf- und Halskrebs, Nierenkrebs, kolorektales Karzinom, multiples Myelom, Leukämie einschließlich akuter Leukämie und Speiseröhrenkarzinom.

## Revendications

1. Protéine de fusion comprenant :
(a) l'antigène du cancer des testicules PRAME ;
(b) une protéine partenaire de fusion hétérologue dérivée de la Protéine D, où la protéine partenaire de fusion comprend les acides aminés 20 à 127 de la Protéine D ; et
(c) les acides aminés supplémentaires Met-Asp-Pro à l'extrémité N-terminale de la séquence de la protéine de fusion,
où la protéine partenaire de fusion hétérologue dérivée de la Protéine D, (b), est liée par fusion à l'extrémité N-terminale de PRAME.

2. Protéine de fusion selon la revendication 1, comprenant en outre une étiquette d'affinité.

3. Protéine de fusion selon la revendication 2, dans laquelle l'étiquette d'affinité est une queue histidine.

4. Protéine de fusion selon l'une quelconque des revendications précédentes, comprenant en outre une ou plusieurs séquences du type lieur entre la protéine partenaire de fusion et PRAME ; ou entre la protéine partenaire de fusion et l'étiquette d'affinité ; ou entre PRAME et l'étiquette d'affinité.

5. Séquence d'acide nucléique codant pour une protéine de fusion selon l'une quelconque des revendications 1 à 4.

6. Vecteur comprenant la séquence d'acide nucléique selon la revendication 5.

7. Cellule hôte cible transformée avec le vecteur selon la revendication 6.

8. Vaccin contenant une protéine de fusion selon l'une quelconque des revendications 1 à 4 ou un acide nucléique selon la revendication 5 ou un vecteur selon la revendication 6.

9. Vaccin selon la revendication 8, comprenant en outre un adjuvant et/ou une cytokine ou chimiokine immunostimulante.

10. Vaccin selon la revendication 9, dans lequel l'adjuvant comprend 3D-MPL, QS21 et/ou un oligonucléotide CpG.

11. Vaccin selon l'une quelconque des revendications 8 à 10, pour une utilisation en médecine.

12. Utilisation d'une protéine ou d'un acide nucléique ou d'un vecteur selon l'une quelconque des revendications 1 à 6, pour la fabrication d'un vaccin pour le traitement en immunothérapie d'un patient atteint de cancer.

13. Procédé de production d'une protéine de fusion comprenant l'étape consistant à exprimer dans une cellule isolée, une protéine de fusion selon l'une quelconque des revendications 1 à 4.

14. Procédé selon la revendication 13, dans lequel la cellule est une bactérie.

15. Procédé selon la revendication 14, dans lequel la bactérie est E. *coli.*

16. Procédé selon la revendication 13, comprenant en outre l'étape consistant à lyser la cellule et à purifier la protéine de fusion exprimée à partir des cellules lysées.

17. Protéine de fusion obtenue ou pouvant être obtenue par le procédé selon l'une quelconque des revendications 13 à 16.

18. Utilisation selon la revendication 12, où le cancer est choisi parmi le mélanome, le cancer du sein, de la vessie, le cancer du poumon tel que le NSCLC, le sarcome, le cancer de l'ovaire, le cancer de la tête et du cou, le cancer du rein, le cancer colorectal, le myélome multiple, la leucémie comprenant la leucémie aiguë et le cancer de l'oesophage.
